# EUROPÄISCHE PATENTSCHRIFT

(11) **EP 0 858 446 B1**
(45) Veröffentlichungstag und Bekanntmachung des Hinweises auf die Patenterteilung: **07.03.2001**
(21) Anmeldenummer: 96934776.4
(22) Anmeldetag: 22.10.1996
(51) Int. Cl.: C07C 271/28, C07C 259/06, C07C 259/08, C07C 275/64, A01N 47/20, A01N 47/30, A01N 37/28

(54) **PHENYLCARBAMATE, VERFAHREN UND ZWISCHENPRODUKTE ZU IHRER HERSTELLUNG UND IHRE VERWENDUNG ZUR BEKÄMPFUNG VON SCHÄDLINGEN UND SCHADPILZEN**
PHENYLCARBAMATES, PROCESSES AND INTERMEDIATE PRODUCTS FOR THEIR PREPARATION AND THEIR USE AS PESTICIDES AND FUNGICIDES
PHENYLCARBAMATES, PROCEDES ET PRODUITS INTERMEDIAIRES PERMETTANT DE LES PREPARER, ET LEUR UTILISATION POUR LUTTER CONTRE DES PARASITES ET DES CHAMPIGNONS NUISIBLES

(30) Priorität: 02.11.1995 DE 19540735
(43) Veröffentlichungstag der Anmeldung: 19.08.1998
(73) Patentinhaber: BASF AKTIENGESELLSCHAFT, 67056 Ludwigshafen (DE)
(72) Erfinder: MÜLLER, Bernd, D-67227 Frankenthal (DE); SAUTER, Hubert, D-68167 Mannheim (DE); BAYER, Herbert, D-68159 Mannheim (DE); GRAMMENOS, Wassilios, D-67063 Ludwigshafen (DE); GROTE, Thomas, D-67105 Schifferstadt (DE); KIRSTGEN, Reinhard, D-67434 Neustadt (DE); OBERDORF, Klaus, D-69117 Heidelberg (DE); RÖHL, Franz, D-67105 Schifferstadt (DE); GÖTZ, Norbert, D-67547 Worms (DE); RACK, Michael, D-69123 Heidelberg (DE); MÜLLER, Ruth, D-67159 Friedelsheim (DE); LORENZ, Gisela, D-67434 Hambach (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(86) Internationale Anmeldenummer: EP9604575
(87) Internationale Veröffentlichungsnummer: WO9716415

(56) Entgegenhaltungen:
- EP-A- 0 619 301
- EP-A- 0 627 411
- EP-A- 0 704 430
- WO-A-93/15046

## Beschreibung

Die vorliegende Erfindung betrifft Phenylcarbamate der Formel I 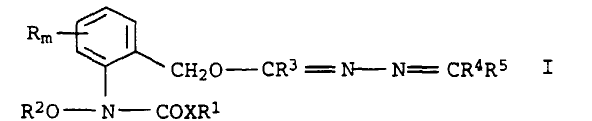 in der die Substituenten und der Index die folgende Bedeutung haben:
- R: Cyano, Nitro, Trifluormethyl, Halogen, C₁-C₄-Alkyl oder C₁-C₄-Alkoxy;
- m: 0, 1 oder 2, wobei die Reste R verschieden sein können, wenn n für 2 steht;
- R¹: Alkyl, Alkenyl, Alkinyl, Cycloalkyl oder Cycloalkenyl, und für den Fall, daß X für NR^{a} steht, zusätzlich Wasserstoff;
- X: eine direkte Bindung, O oder NR^{a};
- R^{a}: Wasserstoff, Alkyl, Alkenyl, Alkinyl, Cycloalkyl oder Cycloalkenyl;
- R²: Wasserstoff,
ggf. subst. Alkyl, Alkenyl, Alkinyl, Cycloalkyl, Cycloalkenyl, Cycloalkinyl, Alkylcarbonyl oder Alkoxycarbonyl;
- R³ und R⁴: unabhängig voneinander
Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können und/oder einen bis drei der folgenden Reste tragen können:
- Cyano, Nitro, Hydroxy, Mercapto, Amino, Carboxyl, Aminocarbonyl, Aminothiocarbonyl,
- C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy,
- C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio und Hetaryl- C₁-C₄-alkylthio, wobei die cyclischen Reste ihrerseits partiell oder vollständig halogeniert sein können und/oder eine bis drei der folgenden Gruppen tragen können: Cyano, Nitro, Hydroxyl, Mercapto, Amino, Carboxyl, Aminocarbonyl, Aminothiocarbonyl, C₁-C₆- Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆- Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR^{b})-Aₙ-R^{c};
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₅-C₈-Cycloalkenyl, C₅-C₈-Cycloalkenyloxy, C₅-C₈-Cycloalkenylthio, C₅-C₈-Cycloalkenylamino, N-C₅-C₈-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Alkoxy-C₁-C₆-alkyl, C₁-C₆-Halogenalkoxy, C₁-C₆-Alkoxycarbonyl, C₁-C₆-Alkylcarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Aryl-C₁-C₆-alkoxy, Aryl, Aryloxy, Hetaryl, Hetaryloxy, wobei die cyclischen Reste der sechs letztgenannten Gruppen partiell oder vollständig halogeniert sein können und/ oder eine C₁-C₆-Alkylgruppe tragen können, C(=NOR^{b})-Aₙ-R^{c} oder NR^{f}-CO-D-R^{g};
- A: Sauerstoff, Schwefel oder Stickstoff, wobei der Stickstoff Wasserstoff oder C₁-C₆-Alkyl trägt;
- D: eine direkte Bindung, Sauerstoff oder NR^{h};
- n: 0 oder 1;
- R^{b} und R^{c}: unabhängig voneinander Wasserstoff oder C₁-C₆-Alkyl;
- R^{f}: Wasserstoff, Hydroxy, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl;
- R^{g}, R^{h}: unabhängig voneinander Wasserstoff, C₁-C₆-Alkyl, C₂-C₆-Alkenyl, C₂-C₆-Alkinyl, C₃-C₆-Cycloalkyl, C₃-C₆-cycloalkenyl, Aryl, Aryl-C₁-C₆-alkyl, Hetaryl und HetarylC₁-C₆-alkyl;
- R⁵: eine Gruppe CR^{d}=NOR^{e};
- R^{d}: eine der bei R³ genannten Gruppen;
- R^{e}: Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₂-C₁₀-Alkinylcarbonyl oder C₁-C₁₀-Alkylsulfonyl, wobei diese Reste partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die letzten zwölf genannten Gruppen ihrerseits partiell oder vollständig halogeniert sein können und/ oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C (=NOR^{b})-Aₙ-R^{c};

Aryl, Arylcarbonyl, Arylsulfonyl, Hetaryl, Hetarylcarbonyl oder Hetarylsulfonyl, wobei diese Reste partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C (=NOR^{b})-Aₙ-R^{c},
sowie deren Salze.

Außerdem betrifft die Erfindung Verfahren und Zwischenprodukte zur Herstellung dieser Verbindungen sowie sie enthaltende Mittel zur Bekämpfung von tierischen Schädlingen und Schadpilzen.

Aus der Literatur sind Anilide zur Bekämpfung von Schadpilzen bekannt (WO-A 93/15046). Außerdem werden in DE-A 44 41 674 (≙ WO 96/16030) Anilide mit Wirkung gegen tierische Schädlinge und Schadpilze beschrieben.

In der nachveröffentlichten EP-A 704 430 werden Anilide mit orthoständiger Azin-Seitenkette und deren fungizide Verwendung beschrieben. Im Gegensatz zu den erfindungsgemäßen Verbindungen weisen die aus EP-A 704 430 bekannten Anilidderivate keine Alkoximinomethyl-Gruppierung am terminalen Kohlenstoff des Azinteils auf.

Der vorliegenden Erfindung lagen demgegenüber Verbindungen mit verbesserter Wirkung als Aufgabe zugrunde.

Demgemäß wurden die eingangs definierten Phenylcarbamate I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung in diesem Sinne gefunden.

Die Verbindungen I sind auf verschiedenen Wegen nach an sich in der Literatur bekannten Verfahren erhältlich.

Grundsätzlich ist es bei der Synthese der Verbindungen I unerheblich, ob zunächst die Gruppierung -N(OR²)-COXR¹ oder die Gruppierung -CH₂OCR³=NN=CR⁴R⁵ aufgebaut wird.

Die Art der Synthese der -CH₂OCR³=NN=CR⁴R⁵ Seitenkette richtet sich im wesentlichen nach der Art des Substituenten R³. Zur besseren Übersichtlichkeit ist in den folgenden Reaktionsschemata die Gruppierung -N(OR²)-COXR¹ bzw. eine geeignete Vorstufe zu dieser Gruppe mit # verkürzt dargestellt.

Bedeutet # -N(OR²)-COXR¹, so handelt es sich bei β um Verbindungen der Formel II und bei α um Verbindungen der Formel I.

Bedeutet # -NHOH, so handelt es sich bei β um L¹-substituiertes ortho-Hydroxyaminotoluol, das durch Rₘ substituiert ist, und bei α um Verbindungen der Formel V. Bedeutet # -NO₂, so handelt es sich bei β um L₁-substituiertes ortho-Nitrotoluol, das durch Rₘ substituiert ist, und bei α um Verbindungen IV.

1.1 Für den Fall, daß R³ nicht Halogen bedeutet, geht man beim Aufbau der -CH₂OCR³=NN=CR⁴R⁵ Seitenkette im allgemeinen so vor, daß man ein Benzylderivat der Formel β mit einem Carbonsäurehydrazid der Formel III umsetzt. 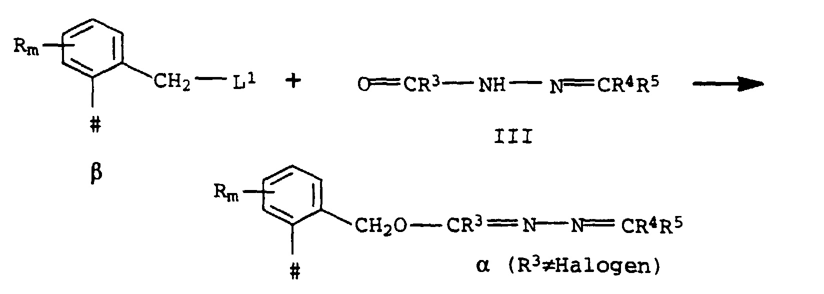 L¹ in der Formel β steht für eine nucleophil austauschbare Abgangsgruppe, z.B. Halogen oder Sulfonatgruppen, vorzugsweise Chlor, Brom, Iod, Mesylat, Tosylat oder Triflat. Diese Umsetzung erfolgt üblicherweise bei Temperaturen von - 10°C bis 80°C, vorzugsweise 0°C bis 50°C in einem inerten organischen Lösungsmittel in Gegenwart einer Base *[vgl. Houben-Weyl, Bd. E 14b, S. 370f und Houben-Weyl, Bd. 10/1, S. 1189f].*

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid, Dimethylformamid und Wasser, besonders bevorzugt Dimethylformamid, Acetonitril, Toluol, tert.-Butylmethylether und Wasser. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-butanolat und Dimethoxymagnesium außerdem organische Basen, z.B. tertiäre Amine wie Trimethylamin, Triethylamin, Tri-isopropylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydrid, Kaliumhydroxyd, Kaliumcarbonat und Triethylamin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, III in einem Überschuß bezogen auf β einzusetzen.

Die für die Herstellung der Verbindungen I benötigten Ausgangsstoffe der Formel II sind in der eingangs zitierten Literatur beschrieben oder können nach den dort beschriebenen Verfahren hergestellt werden.

Die Ausgangsstoffe der Formel III sind ebenfalls aus der Literatur bekannt [*vgl. Tetrahedron 1987, 4185; Houben-Weyl, Register der Stoffklassen Teil A, Bd. 16/2, S. 439f.*] oder können nach den bekannten Verfahren hergestellt werden.

1.2 Verbindungen α, in denen R³ für ein Halogenatom steht, erhält man aus den entsprechenden Vorstufen, in denen der betreffende Rest für eine Hydroxygruppe steht, nach an sich bekannten Verfahren *[vgl. Houben-Weyl, Bd. E 5, S. 631f; J. Org. Chem. 36, 233 (1971); J. Org. Chem. 57, 3245 (1992)].*

1.3 Verbindungen α, in denen R³ über ein O-, S- oder N-Atom an das Molekülgerüst gebunden ist, erhält man aus den entsprechenden Vorstufen, in denen der betreffende Rest für ein Halogenatom steht, nach an sich bekannten Verfahren *[vgl. Houben-Weyl, Bd. E 5, S. 826f; J. Org. Chem. 36*, *233 (1971); J. Org. Chem. 46, 3623 (1981)].*

1.4 Verbindungen α, in denen R³ über ein Sauerstoffatom an das Molekül gebunden ist, erhält man auch durch Veretherung der entsprechenden Vorstufen, in denen der betreffende Rest für eine Hydroxylgruppe steht, nach an sich bekannten Verfahren *[vgl. Houben-Weyl, Bd. E 5, S. 826f; Aust. J. Chem. 27, 1341 (1974)].*

Der Aufbau der Gruppierung -N(OR²)-COXR¹ ist beispielsweise aus der eingangs zitierten Literatur bekannt. Zur besseren Übersichtlichkeit ist in den folgenden Reaktionsschemata die CH₂OCR³=NN=CR⁴R⁵ Seitenkette bzw. eine geeignete Vorstufe zu dieser Gruppe mit * verkürzt dargestellt.

Bedeutet * CH₂OR³=N-N=CR⁴R⁵, so handelt es sich bei δ um Verbindungen der Formel IV, bei ε um Verbindungen der Formel V und bei η um Verbindungen der Formel I.

Bedeutet * CH₂L¹, so handelt es sich bei δ um L¹-substituiertes ortho-Nitrotoluol, das durch Rₘ substituiert ist, bei ε um L¹-substituiertes ortho-Hydroxyaminotoluol, das durch Rₘ substituiert ist und bei η um ein L¹-substituiertes N-Acyl-(hydroxyamino)toluol, das durch Rₘ substituiert ist.

Bedeutet * CH₃, so handelt es sich bei δ um ein ortho-Nitrotoluol, das durch Rₘ substituiert ist, bei ε um ein ortho-Hydroxyaminotoluol, das durch Rₘ-substituiert ist, und bei η um ein N-Acyl-(hydroxyamino)toluol, das durch Rₘ substituiert ist.

2.1 Verbindungen η, in denen R² Wasserstoff bedeutet (IA), erhält man im allgemeinen dadurch, daß man ein Nitrobenzol der Formel δ zum entsprechenden Hydroxylamin ε reduziert und ε anschließend durch Umsetzung mit einem Acylierungsmittel der Formel VI zu η (mit R²=H) umsetzt. 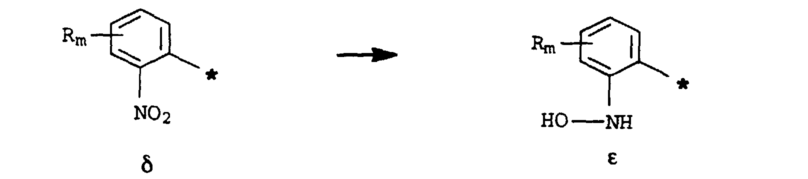 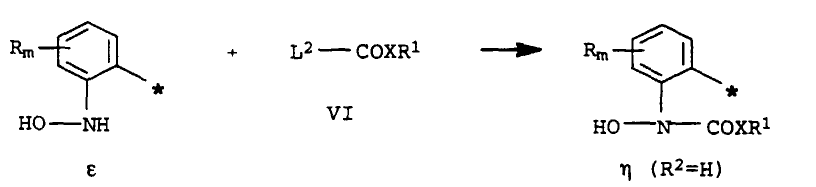 L² in der Formel VI steht beispielsweise für Halogen oder Aryloxy, insbesondere Chlor und Phenoxy.
a) Die Reduktion von δ zum Hydroxylamin ε erfolgt üblicherweise bei Temperaturen von -30°C bis 80°C, vorzugsweise 0°C bis 60°C in einem inerten organischen Lösungsmittel in Gegenwart eines Katalysators *[vgl. DE Anm. Nr. 19 50 27 00.0].*
b) Die Umsetzung von den Hydroxylamins ε mit VI erfolgt üblicherweise bei Temperaturen von -20°C bis 60°C, vorzugsweise 0°C bis 30°C in einem inerten organischen Lösungsmittel in Gegenwart einer Base *[vgl. WO-A 93/15046].*

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid, Dimethylformamid und Wasser, besonders bevorzugt Cyclohexan, Toluol, Methylenchlorid, tert.-Butylmethylether und Wasser. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-butanolat und Dimethoxymagnesium außerdem organische Basen, z.B. tertiäre Amine wie Trimethylamin, Triethylamin, Triisopropylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydroxyd und Triethylamin.

Die Basen werden im allgemeinen in katalytischen Mengen eingesetzt, sie können aber auch äquimolar, im Überschuß oder gegebenenfalls als Lösungsmittel verwendet werden.

2.2 Die Verbindungen η, in denen R² nicht Wasserstoff bedeutet (IB), erhält man dadurch, daß man eine Verbindungen der Formel η mit R² = H in an sich bekannter Weise mit einer Verbindung der Formel VII umsetzt. 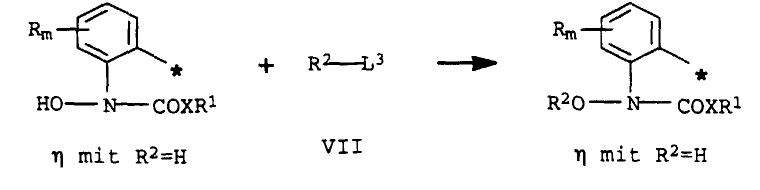 L³ in der Formel VII bedeutet beispielsweise Halogen, Mesylat, Tosylat, Carboxylat und Sulfat, insbesondere Chlor, Brom, Mesylat und R²-OSO₃-.

Diese Umsetzung erfolgt üblicherweise bei Temperaturen von -20°C bis 80°C, vorzugsweise 0°C bis 60°C in einem inerten organischen Lösungsmittel in Gegenwart einer Base *[vgl. WO-A 93/15046].*

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Aceton, Toluol, tert.-Butylmethylether, Cyclohexan und Wasser. Es können auch Gemische der genannten Lösungsmittel verwendet werden.

Als **Basen** kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-butanolat und Dimethoxymagnesium außerdem organische Basen, z.B. tertiäre Amine wie Trimethylamin, Triethylamin, Triisopropylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydroxyd und Triethylamin.

Die Basen werden im allgemeinen in katalytischen Mengen eingesetzt, sie können aber auch äquimolar, im Überschuß oder gegebenenfalls als Lösungsmittel verwendet werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die -CR³=N-N=CR⁴R⁵ Doppelbindungen werden im allgemeinen hinsichtlich ihrer Wirksamkeit die cis-Isomere der Verbindungen I (Konfiguration bezogen auf den Rest R³ im Verhältnis zur -N=CR⁴R⁵-Gruppe bzw. bezogen auf den Rest R⁴ im Verhältnis zur -N=CR³-Gruppe) bevorzugt.

Die Verbindungen I können saure oder basische Zentren enthalten und dementsprechend Säureadditionsprodukte oder Basenadditionsprodukte oder Salze bilden.

Säuren für Säureadditionsprodukte sind u.a. Mineralsäuren (z.B. Halogenwasserstoffsäuren wie Chlorwasserstoff- und Bromwasserstoffsäure, Phosphorsäure, Schwefelsäure, Salpetersäure), organische Säuren (z.B. Ameisensäure, Essigsäure, Oxalsäure, Malonsäure, Milchsäure, Äpfelsäure, Bernsteinsäure, Weinsäure, Zitronensäure, Salicylsäure, p-Toluolsulfonsäure, Dodecylbenzolsulfonsäure) oder andere protonenacide Verbindungen (z.B. Saccharin).

Basen für Basenadditionsprodukte sind u.a. Oxide, Hydroxide, Carbonate oder Hydrogencarbonate von Alkalimetallen oder Erdalkalimetallen (z.B. Kalium- oder Natriumhydroxyd oder -carbonat) oder Ammoniumverbindungen (z.B. Ammoniumhydroxyd).

Bei der eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl**: geradkettige oder verzweigte Alkylgruppen mit 1 bis 10 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, trägt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 10 Kohlenstoffatomen, welche über eine Sulfonylgruppe (-SO₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlor fluormethyl, Dichlorfluormethyl, Chlordifluormethyl, 1-Fluorethyl, 2-Fluorethyl, 2,2-Di-fluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methylpropyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind;
**Alkylthio:** geradkettige oder verzweigte Alkylgruppen mit 1 bis 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
**Cycloalkyl:** monocyclische Alkylgruppen mit 3 bis 6 Kohlenstoffringgliedern, z.B. Cyclopropyl, Cyclobutyl, Cyclopentyl und Cyclohexyl;
**Alkenyl:** geradkettige oder verzweigte Alkenylgruppen mit 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-l-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, 1-Ethyl-2-methyl-1-propenyl und 1-Ethyl-2-methyl-2-propenyl;
**Alkenyloxy:** geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylthio bzw. Alkenylamino:** geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoffatom an das Gerüst gebunden sind.
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkinyl:** geradkettige oder verzweigte Alkinylgruppen mit 3 bis 10 Kohlenstoffatomen und einer Dreifachbindung in einer beliebigen Position, z.B. C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind.
**Alkinylcarbonyl:** geradkettige oder verzweigte Alkinylgruppen mit 3 bis 10 Kohlenstoffatomen und einer Dreifachbindung in einer beliebigen Position, welche über eine Carbonylgruppe (-CO-) an das Gerüst gebunden sind;
**Cycloalkenyl bzw. Cycloalkenyloxy, Cycloalkenylthio und Cycloalkenylamino:** monocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder Cycloalkenylamino) über ein Stickstoffatom an das Gerüst gebunden sind, z.B. Cyclobutenyl, Cyclopentenyl oder Cyclohexenyl.
**Cycloalkoxy bzw. Cycloalkylthio und Cycloalkylamino:** monocyclische Alkylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl oder Cyclohexyl;
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 5-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,2,4-Triazolidin-5-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydrofur-4-yl, 2,3-Dihydrofur-5-yl, 2,5-Dihydrofur-2-yl, 2,5-Dihydrofur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,3-Dihydroisothiazol-3-yl, 2,3-Dihydroisothiazol-4-yl, 2,3-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5,Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl;
**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Arylamino:** aromatische mono- oder polycyclische Kohlenwasserstoffreste, welche über ein Stickstoffatom an das Gerüst gebunden sind.
**Hetaryl bzw. Hetaryloxy, Hetarylthio, Hetarylcarbonyl und Hetarylsulfonyl:** aromatische mono- oder polycyclische Reste welche neben Kohlenstoffringgliedern zusätzlich ein bis vier Stickstoffatome, oder ein bis drei Stickstoffatome und ein Sauerstoff- oder ein Schwefelatom, oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.
   - 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 5-Imidazolyl, 1,2,4-Triazol-3-yl, 1,2,4-Triazol-5-yl, 1,2,3-Triazolyl-4-yl, 1,2,3-Triazol-5-yl, 1,3,4-Triazol-2-yl und 1,3,4-Triazol-5-yl;
   - 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom, oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,2,4-Triazol-5-yl, 1,3,4-Oxadiazol-2-yl, 1,3,4-Oxadiazol-5-yl, 1,3,4-Thiadiazol-2-yl, 1,3,4-Thiadiazol-5-yl, 1,3,4-Triazol-2-yl und 1,3,4-Triazol-5-yl;
   - benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome, oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
   - benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin, bzw. die entsprechenden Oxy-, Thio-, Carbonyl- oder Sulfonylgruppen.
**Hetarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom enthalten können und welche über ein Stickstoffatom an das Gerüst gebunden sind.

Die Angabe *"partiell oder vollständig halogeniert"* soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Die Angabe *"ggf*. *subst."* in Bezug auf die bei der Definition von R² genannten Reste soll zum Ausdruck bringen, daß die betreffenden Gruppen partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkylcarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C(=NOR^{b})-Aₙ-R^{c} oder durch übliche Gruppen substituiertes Benzyloxy und Benzylthio;
- C₃-C₆-Cycloalkyl, C₃-C₆-Cycloalkoxy, Heterocyclyl, Heterocyclyloxy, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder eine bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C(=NOR^{b})-Aₙ-R^{c}, oder durch übliche Gruppen substituiertes Benzyl, Benzyloxy, Aryl, Aryloxy, Arylthio, Hetaryl, Hetaryloxy und Hetarylthio.

Die Angabe *"durch übliche Gruppen substituiert"* soll zum Ausdruck bringen, daß die betreffenden Reste partiell oder vollständig halogeniert sein können und/oder einen bis drei der folgenden Substituenten tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylcarbonyl, C₁-C₄-Alkoxycarbonyl, Phenyl, Phenoxy, Benzyl und Benzyloxy, und/oder eine Gruppe C(=NOR^{b})-Aₙ-R^{c} tragen können.

Im Hinblick auf ihre biologische Wirkung sind Verbindungen der Formel I hervorzuheben, in der die Substituenten und der Index die folgende Bedeutung haben:
- R: Cyano, Nitro, Trifluormethyl, Halogen, C₁-C₄-Alkyl oder C₁-C₄-Alkoxy;
- m: 0, 1 oder 2, wobei die Reste R verschieden sein können, wenn n für 2 steht;
- R¹: Alkyl, Alkenyl, Alkinyl, Cycloalkyl oder Cycloalkenyl, und für den Fall, daß X für NR^{a} steht, zusätzlich Wasserstoff;
- X: eine direkte Bindung, O oder NR^{a};
- R^{a}: Wasserstoff, Alkyl, Alkenyl, Alkinyl, Cycloalkyl oder Cycloalkenyl;
- R²: Wasserstoff,
ggf. subst. Alkyl, Alkenyl, Alkinyl, Cycloalkyl, Cycloalkenyl, Cycloalkinyl, Alkylcarbonyl oder Alkoxycarbonyl;
- R³ und R⁴: unabhängig voneinander
Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können und/oder einen bis drei der folgenden Reste tragen können:
- Cyano, Nitro, Hydroxy, Mercapto, Amino, Carboxyl, Aminocarbonyl, Aminothiocarbonyl,
- C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy,
- C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio und Hetaryl-C₁-C₄-alkylthio, wobei die cyclischen Reste ihrerseits partiell oder vollständig halogeniert sein können und/ oder eine bis drei der folgenden Gruppen tragen können: Cyano, Nitro, Hydroxyl, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆- Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR^{b})-Aₙ-R^{c};
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₅-C₈-Cycloalkenyl, C₅-C₈-Cycloalkenyloxy, C₅-C₈-Cycloalkenylthio, C₅-C₈-Cycloalkenylamino, N-C₅-C₈-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{b})-Aₙ-R^{c} oder NR^{f}-CO-D-R^{g};
- A: Sauerstoff, Schwefel oder Stickstoff, wobei der Stickstoff Wasserstoff oder C₁-C₆-Alkyl trägt;
- D: eine direkte Bindung, Sauerstoff oder NR^{h};
- n: 0 oder 1;
- R^{b} und R^{c}: unabhängig voneinander Wasserstoff oder C₁-C₆-Alkyl;
- R^{f}: Wasserstoff, Hydroxy, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl;
- R^{g}, R^{h}: unabhängig voneinander Wasserstoff, C₁-C₆-Alkyl, C₂-C₆-Alkenyl, C₂-C₆-Alkinyl, C₃-C₆-Cycloalkyl, C₃-C₆-Cycloalkenyl, Aryl, Aryl-C₁-C₆-alkyl, Hetaryl und Hetaryl-C₁-C₆-alkyl;
- R⁵: eine Gruppe CR^{d}=NOR^{e};
- R^{d}: eine der bei R³ genannten Gruppen;
- R^{e}: Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₂-C₁₀-Alkinylcarbonyl oder C₁-C₁₀-Alkylsulfonyl, wobei diese Reste partiell oder vollständig halogeniert sein können und/ oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die letzten zwölf genannten Gruppen ihrerseits partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C (=NOR^{b})-Aₙ-R^{c};
Aryl, Arylcarbonyl, Arylsulfonyl, Hetaryl, Hetarylcarbonyl oder Hetarylsulfonyl, wobei diese Reste partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR^{b})-Aₙ-R^{c},
sowie deren Salze.

Im Hinblick auf ihre biologische Wirkung sind Verbindungen der Formel I bevorzugt, in denen m für 0 oder 1, insbesondere für 0, steht.

Für den Fall, daß m nicht 0 bedeutet, werden Verbindungen I bevorzugt, in denen R für Fluor, Chlor, Methyl und Trifluormethyl steht.

Insbesondere werden Verbindungen I bevorzugt, in denen X für Sauerstoff steht.

Außerdem werden Verbindungen I besonders bevorzugt, in denen X für NH steht.

Gleichermaßen besonders bevorzugt sind Verbindungen I, in denen X für eine direkte Bindung steht.

Insbesondere werden Verbindungen I bevorzugt, in denen R¹ für Methyl steht.

Außerdem werden Verbindungen I besonders bevorzugt, in denen R1 für Ethyl steht.

Gleichermaßen besonders bevorzugt sind Verbindungen I, in denen R¹ für Cyclopropyl steht.

Daneben werden Verbindungen I besonders bevorzugt, in denen R¹ für Wasserstoff steht (X steht für NR^{a}).

Insbesondere werden Verbindungen I bevorzugt, in denen R² für Wasserstoff steht.

Außerdem werden Verbindungen I besonders bevorzugt, in denen R² für Methyl steht.

Gleichermaßen besonders bevorzugt sind Verbindungen I, in denen R² für Ethyl steht.

Daneben werden Verbindungen I besonders bevorzugt, in denen R² für Methoxymethyl, Allyl oder Propargyl steht.

Insbesondere werden Verbindungen I bevorzugt, in denen R³ für C₁-C₄-Alkyl, besonders Methyl, steht.

Außerdem werden Verbindungen I besonders bevorzugt, in denen R³ für Aryl, besonders ggf. subst. Phenyl, steht.

Gleichermaßen besonders bevorzugt sind Verbindungen I, in denen R³ für Hetaryl, besonders ggf. subst. Isoxazolyl, Pyrazolyl und Pyridinyl, steht.

Daneben werden Verbindungen I besonders bevorzugt, in denen R³ für ggf. subst. Cycloalkyl, besonders Cycloalkyl, steht.

Insbesondere werden Verbindungen I bevorzugt, in denen R⁴ für C₁-C₄-Alkyl, besonders Methyl, steht.

Außerdem werden Verbindungen I besonders bevorzugt, in denen R⁴ für Aryl, besonders ggf. subst. Phenyl, steht.

Gleichermaßen besonders bevorzugt sind Verbindungen I, in denen R⁴ für ggf. subst. Cycloalkyl, besonders Cycloalkyl, steht.

Daneben werden Verbindungen I besonders bevorzugt, in denen R⁴ für Hetaryl, besonders ggf. subst. Isoxazolyl, Pyrazolyl und Pyridinyl, steht.

Erfindungsgemäß sind Verbindungen I, in denen R⁵ für eine Gruppe CR^{d}=NOR^{e} steht.

Insbesondere werden Verbindungen I bevorzugt, in denen R^{d} für C₁-C₄-Alkyl, besonders Methyl, steht.

Insbesondere werden Verbindungen I bevorzugt, in denen R^{d} für Aryl, besonders ggf. subst. Phenyl, steht.

Außerdem werden Verbindungen I besonders bevorzugt, in denen R^{d} für Hetaryl, besonders ggf. subst. Isoxazolyl, Pyrazolyl und Pyridinyl, steht.

Gleichermaßen besonders bevorzugt sind Verbindungen I, in denen R^{d} für ggf. subst. Cycloalkyl, besonders Cycloalkyl, steht.

Daneben werden Verbindungen I besonders bevorzugt, in denen R^{e} für C₁-C₄-Alkyl, besonders Methyl, steht.

Außerdem werden Verbindungen I besonders bevorzugt, in denen R^{e} für ggf. subst. Alkenyl, besonders Allyl, steht.

Gleichermaßen besonders bevorzugt sind Verbindungen I, in denen R^{e} für ggf. subst. Alkinyl, besonders Propargyl, steht.

Daneben werden Verbindungen I besonders bevorzugt, in denen R^{e} für Methoxyethyl steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der allgemeinen Formel IC (m = 0), in denen XR¹ Methoxy, R² Wasserstoff, R³ Methyl, R^{d} Methyl und R^{e} Methyl bedeutet und R⁴ für durch R^{x} substituiertes Phenyl steht, wobei R^{x} jeweils einer Zeile der Tabelle B entspricht.

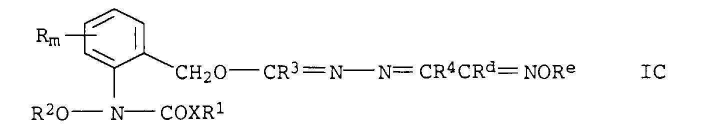

### Tabelle 2

Verbindungen der allgemeinen Formel IC (m = 0), in denen XR¹ Methoxy, R² Methyl, R³ Methyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 3

Verbindungen der allgemeinen Formel IC (m = 0), in denen XR¹ Methylamino, R² Wasserstoff, R³ Methyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 4

Verbindungen der allgemeinen Formel IC (m = 0), in denen XR¹ Methylamino, R² Methyl, R³ Methyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 5

Verbindungen der allgemeinen Formel IC (m = 0), in denen XR¹ Methoxy, R² Wasserstoff, R³ Ethyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 6

Verbindungen der allgemeinen Formel IC (m = 0), in denen XR¹ Methoxy, R² Ethyl, R³ Ethyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 7

Verbindungen der allgemeinen Formel IC (m = 0), in denen XR¹ Methylamino, R² Wasserstoff, R³ Ethyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 8

Verbindungen der allgemeinen Formel IC (m = 0), in denen XR¹ Methylamino, R² Ethyl, R³ Ethyl, R^{d} Methyl und R^{e} Methyl bedeutet und R⁴ für durch R^{x} substituiertes Phenyl steht, wobei R^{x} für eine Verbindung jeweils einer Zeile der Tabelle B entspricht

### Tabelle 9

Verbindungen der allgemeinen Formel IC (m = 0), in denen XR¹ Methoxy, R² Wasserstoff, R³ Cyclopropyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 10

Verbindungen der allgemeinen Formel IC (m = 0), in denen XR¹ Methoxy, R² Cyclopropyl, R³ Cyclopropyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 11

Verbindungen der allgemeinen Formel IC (m = 0), in denen XR¹ Methylamino, R² Wasserstoff, R³ Cyclopropyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 12

Verbindungen der allgemeinen Formel IC (m = 0), in denen XR¹ Methylamino, R² Cyclopropyl, R³ Cyclopropyl, R^{d} Methyl und R^{e} Methyl bedeutet und R⁴ für durch R^{x} substituiertes Phenyl steht, wobei R^{x} jeweils einer Zeile der Tabelle B entspricht.

### Tabelle 13

Verbindungen der allgemeinen Formel IC (m = 0), in denen XR¹ Methoxy, R² Wasserstoff, R³ Methyl, R⁴ Methyl und R^{e} Methyl bedeutet und R^{d} für durch R^{x} substituiertes Phenyl steht, wobei R^{x} jeweils einer Zeile der Tabelle B entspricht.

### Tabelle 14

Verbindungen der allgemeinen Formel IC (m = 0), in denen XR¹ Methoxy, R² Methyl, R³ Methyl, R⁴ Methyl und R^{e} Methyl bedeutet und R^{d} für durch R^{x} substituiertes Phenyl steht, wobei R^{x} jeweils einer Zeile der Tabelle B entspricht.

### Tabelle 15

Verbindungen der allgemeinen Formel IC (m = 0), in denen XR¹ Methylamino, R² Wasserstoff, R³ Methyl, R⁴ Methyl und R^{e} Methyl bedeutet und R^{d} für durch R^{x} substituiertes Phenyl steht, wobei R^{x} jeweils einer Zeile der Tabelle B entspricht.

### Tabelle 16

Verbindungen der allgemeinen Formel IC (m = 0), in denen XR¹ Methylamino, R² Methyl, R³ Methyl, R⁴ Methyl und R^{e} Methyl bedeutet und R^{d} für durch R^{x} substituiertes Phenyl steht, wobei R^{x} jeweils einer Zeile der Tabelle B entspricht.

### Tabelle 17

Verbindungen der allgemeinen Formel IC (m = 0), in denen XR¹ Methoxy, R² Wasserstoff, R³ Methyl, R⁴ Ethyl und R^{e} Methyl bedeutet und R^{d} für durch R^{x} substituiertes Phenyl steht, wobei R^{x} jeweils einer Zeile der Tabelle B entspricht.

### Tabelle 18

Verbindungen der allgemeinen Formel IC (m = 0), in denen XR¹ Methoxy, R² Methyl, R³ Methyl, R⁴ Ethyl und R^{e} Methyl bedeutet und R^{d} für durch R^{x} substituiertes Phenyl steht, wobei R^{x} jeweils einer Zeile der Tabelle B entspricht.

### Tabelle 19

Verbindungen der allgemeinen Formel IC (m = 0), in denen XR¹ Methylamino, R² Wasserstoff, R³ Methyl, R⁴ Ethyl und R^{e} Methyl bedeutet und R^{d} für durch R^{x} substituiertes Phenyl steht, wobei R^{x} jeweils einer Zeile der Tabelle B entspricht.

### Tabelle 20

Verbindungen der allgemeinen Formel IC (m = 0), in denen XR¹ Methylamino, R² Methyl, R³ Methyl, R⁴ Ethyl und R^{e} Methyl bedeutet und R^{d} für durch R^{x} substituiertes Phenyl steht, wobei R^{x} jeweils einer Zeile der Tabelle B entspricht.

### Tabelle 21

Verbindungen der allgemeinen Formel IC (m = 0), in denen XR¹ Methoxy, R² Wasserstoff, R³ Methyl, R⁴ iso-Propyl und R^{e} Methyl bedeutet und R^{d} für durch R^{x} substituiertes Phenyl steht, wobei R^{x} jeweils einer Zeile der Tabelle B entspricht.

### Tabelle 22

Verbindungen der allgemeinen Formel IC (m = 0), in denen XR¹ Methoxy, R² Methyl, R³ Methyl, R⁴ iso-Propyl und R^{e} Methyl bedeutet und R^{d} für durch R^{x} substituiertes Phenyl steht, wobei R^{x} jeweils einer Zeile der Tabelle B entspricht.

### Tabelle 23

Verbindungen der allgemeinen Formel IC (m = 0), in denen XR¹ Methylamino, R² Wasserstoff, R³ Methyl, R⁴ iso-Propyl und R^{e} Methyl bedeutet und R^{d} für durch R^{x} substituiertes Phenyl steht, wobei R^{x} jeweils einer Zeile der Tabelle B entspricht.

### Tabelle 24

Verbindungen der allgemeinen Formel IC (m = 0), in denen XR¹ Methylamino, R² Methyl, R³ Methyl, R⁴ iso-Propyl und R^{e} Methyl bedeutet und R^{d} für durch R^{x} substituiertes Phenyl steht, wobei R^{x} jeweils einer Zeile der Tabelle B entspricht.

### Tabelle 25

Verbindungen der allgemeinen Formel IC (m = 0), in denen XR¹ Methoxy, R² Wasserstoff, R³ Methyl, R⁴ Methyl und R^{e} Ethyl bedeutet und R^{d} für durch R^{x} substituiertes Phenyl steht, wobei R^{x} jeweils einer Zeile der Tabelle B entspricht.

### Tabelle 26

Verbindungen der allgemeinen Formel IC (m = 0), in denen XR¹ Methoxy, R² Methyl, R³ Methyl, R⁴ Methyl und R^{e} Ethyl bedeutet und R^{d} für durch R^{x} substituiertes Phenyl steht, wobei R^{x} jeweils einer Zeile der Tabelle B entspricht.

### Tabelle 27

Verbindungen der allgemeinen Formel IC (m = 0), in denen XR¹ Methylamino, R² Wasserstoff, R³ Methyl, R⁴ Methyl und R^{e} Ethyl bedeutet und R^{d} für durch R^{x} substituiertes Phenyl steht, wobei R^{x} jeweils einer Zeile der Tabelle B entspricht.

### Tabelle 28

Verbindungen der allgemeinen Formel IC (m = 0), in denen XR¹ Methylamino, R² Methyl, R³ Methyl, R⁴ Methyl und R^{e} Ethyl bedeutet und R^{d} für durch R^{x} substituiertes Phenyl steht, wobei R^{x} jeweils einer Zeile der Tabelle B entspricht.

### Tabelle 29

Verbindungen der allgemeinen Formel IC (m = 0), in denen XR¹ Methoxy, R² Wasserstoff, R³ Methyl, R⁴ Methyl und R^{e} n-Propyl bedeutet und R^{d} für durch R^{x} substituiertes Phenyl steht, wobei R^{x} jeweils einer Zeile der Tabelle B entspricht.

### Tabelle 30

Verbindungen der allgemeinen Formel IC (m = 0), in denen XR¹ Methoxy, R² Methyl, R³ Methyl, R⁴ Methyl und R^{e} n-Propyl bedeutet und R^{d} für durch R^{x} substituiertes Phenyl steht, wobei R^{x} jeweils einer Zeile der Tabelle B entspricht.

### Tabelle 31

Verbindungen der allgemeinen Formel IC (m = 0), in denen XR¹ Methylamino, R² Wasserstoff, R³ Methyl, R⁴ Methyl und R^{e} n-Propyl bedeutet und R^{d} für durch R^{x} substituiertes Phenyl steht, wobei R^{x} jeweils einer Zeile der Tabelle B entspricht.

### Tabelle 32

Verbindungen der allgemeinen Formel IC (m = 0), in denen XR¹ Methylamino, R² Methyl, R³ Methyl, R⁴ Methyl und R^{e} n-Propyl bedeutet und R^{d} für durch R^{x} substituiertes Phenyl steht, wobei R^{x} jeweils einer Zeile der Tabelle B entspricht.

### Tabelle 33

Verbindungen der allgemeinen Formel IC (m = 0), in denen XR¹ Methoxy, R² Wasserstoff, R³ Methyl, R⁴ Methyl und R^{e} iso-Propyl bedeutet und R^{d} für durch R^{x} substituiertes Phenyl steht, wobei R^{x} jeweils einer Zeile der Tabelle B entspricht.

### Tabelle 34

Verbindungen der allgemeinen Formel IC (m = 0), in denen XR¹ Methoxy, R² Methyl, R³ Methyl, R⁴ Methyl und R^{e} iso-Propyl bedeutet und R^{d} für durch R^{x} substituiertes Phenyl steht, wobei R^{x} jeweils einer Zeile der Tabelle B entspricht.

### Tabelle 35

Verbindungen der allgemeinen Formel IC (m = 0), in denen XR¹ Methylamino, R² Wasserstoff, R³ Methyl, R⁴ Methyl und R^{e} iso-Propyl bedeutet und R^{d} für durch R^{x} substituiertes Phenyl steht, wobei R^{x} jeweils einer Zeile der Tabelle B entspricht.

### Tabelle 36

Verbindungen der allgemeinen Formel IC (m = 0), in denen XR¹ Methylamino, R² Methyl, R³ Methyl, R⁴ Methyl und R^{e} iso-Propyl bedeutet und R^{d} für durch R^{x} substituiertes Phenyl steht, wobei R^{x} jeweils einer Zeile der Tabelle B entspricht.

### Tabelle 37

Verbindungen der allgemeinen Formel IC (m = 0), in denen XR¹ Methoxy, R² Wasserstoff, R³ Methyl, R⁴ Methyl und R^{e} tert.-Butyl bedeutet und R^{d} für durch R^{x} substituiertes Phenyl steht, wobei R^{x} jeweils einer Zeile der Tabelle B entspricht.

### Tabelle 38

Verbindungen der allgemeinen Formel IC (m = 0), in denen XR¹ Methoxy, R² Methyl, R³ Methyl, R⁴ Methyl und R^{e} tert.-Butyl bedeutet und R^{d} für durch R^{x} substituiertes Phenyl steht, wobei R^{x} jeweils einer Zeile der Tabelle B entspricht.

### Tabelle 39

Verbindungen der allgemeinen Formel IC (m = 0), in denen XR¹ Methylamino, R² Wasserstoff, R³ Methyl, R⁴ Methyl und R^{e} tert.-Butyl bedeutet und R^{d} für durch R^{x} substituiertes Phenyl steht, wobei R^{x} jeweils einer Zeile der Tabelle B entspricht.

### Tabelle 40

Verbindungen der allgemeinen Formel IC (m = 0), in denen XR¹ Methylamino, R² Methyl, R³ Methyl, R⁴ Methyl und R^{e} tert.-Butyl bedeutet und R^{d} für durch R^{x} substituiertes Phenyl steht, wobei R^{x} jeweils einer Zeile der Tabelle B entspricht.

### Tabelle 41

Verbindungen der allgemeinen Formel IC (m = 0), in denen XR¹ Methoxy, R² Wasserstoff, R³ Methyl, R⁴ Methyl und R^{e} Benzyl bedeutet und R^{d} für durch R^{x} substituiertes Phenyl steht, wobei R^{x} jeweils einer Zeile der Tabelle B entspricht.

### Tabelle 42

Verbindungen der allgemeinen Formel IC (m = 0), in denen XR¹ Methoxy, R² Methyl, R³ Methyl, R⁴ Methyl und R^{e} Benzyl bedeutet und R^{d} für durch R^{x} substituiertes Phenyl steht, wobei R^{x} jeweils einer Zeile der Tabelle B entspricht.

### Tabelle 43

Verbindungen der allgemeinen Formel IC (m = 0), in denen XR¹ Methylamino, R² Wasserstoff, R³ Methyl, R⁴ Methyl und R^{e} Benzyl bedeutet und R^{d} für durch R^{x} substituiertes Phenyl steht, wobei R^{x} jeweils einer Zeile der Tabelle B entspricht.

### Tabelle 44

Verbindungen der allgemeinen Formel IC (m = 0), in denen XR¹ Methylamino, R² Methyl, R³ Methyl, R⁴ Methyl und R^{e} Benzyl bedeutet und R^{d} für durch R^{x} substituiertes Phenyl steht, wobei R^{x} jeweils einer Zeile der Tabelle B entspricht.

### Tabelle 45

Verbindungen der allgemeinen Formel IC (m = 0), in denen XR¹ Methoxy, R² Wasserstoff, R³ Methyl, R⁴ Methyl und R^{e} Propargyl bedeutet und R^{d} für durch R^{x} substituiertes Phenyl steht, wobei R^{x} jeweils einer Zeile der Tabelle B entspricht.

### Tabelle 46

Verbindungen der allgemeinen Formel IC (m = 0), in denen XR¹ Methoxy, R² Methyl, R³ Methyl, R⁴ Methyl und R^{e} Propargyl bedeutet und R^{d} für durch R^{x} substituiertes Phenyl steht, wobei R^{x} jeweils einer Zeile der Tabelle B entspricht.

### Tabelle 47

Verbindungen der allgemeinen Formel IC (m = 0), in denen XR¹ Methylamino, R² Wasserstoff, R³ Methyl, R⁴ Methyl und R^{e} Propargyl bedeutet und R^{d} für durch R^{x} substituiertes Phenyl steht, wobei R^{x} jeweils einer Zeile der Tabelle B entspricht.

### Tabelle 48

Verbindungen der allgemeinen Formel IC (m = 0), in denen XR¹ Methylamino, R² Methyl, R³ Methyl, R⁴ Methyl und R^{e} Propargyl bedeutet und R^{d} für durch R^{x} substituiertes Phenyl steht, wobei R^{x} jeweils einer Zeile der Tabelle B entspricht.

### Tabelle 49

Verbindungen der allgemeinen Formel IC (m = 0), in denen XR¹ Methoxy, R² Wasserstoff, R³ Methyl, R⁴ Methyl und R^{e} Brompropargyl bedeutet und R^{d} für durch R^{x} substituiertes Phenyl steht, wobei R^{x} jeweils einer Zeile der Tabelle B entspricht.

### Tabelle 50

Verbindungen der allgemeinen Formel IC (m = 0), in denen XR¹ Methoxy, R² Methyl, R³ Methyl, R⁴ Methyl und R^{e} Brompropargyl bedeutet und R^{d} für durch R^{x} substituiertes Phenyl steht, wobei R^{x} jeweils einer Zeile der Tabelle B entspricht.

### Tabelle 51

Verbindungen der allgemeinen Formel IC (m = 0), in denen XR¹ Methylamino, R² Wasserstoff, R³ Methyl, R⁴ Methyl und R^{e} Brompropargyl bedeutet und R^{d} für durch R^{x} substituiertes Phenyl steht, wobei R^{x} jeweils einer Zeile der Tabelle B entspricht.

### Tabelle 52

Verbindungen der allgemeinen Formel IC (m = 0), in denen XR¹ Methylamino, R² Methyl, R³ Methyl, R⁴ Methyl und R^{e} Brompropargyl bedeutet und R^{d} für durch R^{x} substituiertes Phenyl steht, wobei R^{x} jeweils einer Zeile der Tabelle B entspricht.

### Tabelle 53

Verbindungen der allgemeinen Formel IC (m = 0), in denen XR¹ Methoxy, R² Wasserstoff, R³ Methyl, R⁴ Methyl und R^{e} Iodpropargyl bedeutet und R^{d} für durch R^{x} substituiertes Phenyl steht, wobei R^{x} jeweils einer Zeile der Tabelle B entspricht.

### Tabelle 54

Verbindungen der allgemeinen Formel IC (m = 0), in denen XR¹ Methoxy, R² Methyl, R³ Methyl, R⁴ Methyl und R^{e} Iodpropargyl bedeutet und R^{d} für durch R^{x} substituiertes Phenyl steht, wobei R^{x} jeweils einer Zeile der Tabelle B entspricht.

### Tabelle 55

Verbindungen der allgemeinen Formel IC (m = 0), in denen XR¹ Methylamino, R² Wasserstoff, R³ Methyl, R⁴ Methyl und R^{e} Iodpropargyl bedeutet und R^{d} für durch R^{x} substituiertes Phenyl steht, wobei R^{x} jeweils einer Zeile der Tabelle B entspricht.

### Tabelle 56

Verbindungen der allgemeinen Formel IC (m = 0), in denen XR¹ Methylamino, R² Methyl, R³ Methyl, R⁴ Methyl und R^{e} Iodpropargyl bedeutet und R^{d} für durch R^{x} substituiertes Phenyl steht, wobei R^{x} jeweils einer Zeile der Tabelle B entspricht.

### Tabelle 57

Verbindungen der allgemeinen Formel IC (m = 0), in denen XR¹ Methoxy, R² Wasserstoff, R³ Methyl, R⁴ Methyl und R^{e} Allyl bedeutet und R^{d} für durch R^{x} substituiertes Phenyl steht, wobei R^{x} jeweils einer Zeile der Tabelle B entspricht.

### Tabelle 58

Verbindungen der allgemeinen Formel IC (m = 0), in denen XR¹ Methoxy, R² Methyl, R³ Methyl, R⁴ Methyl und R^{e} Allyl bedeutet und R^{d} für durch R^{x} substituiertes Phenyl steht, wobei R^{x} jeweils einer Zeile der Tabelle B entspricht.

### Tabelle 59

Verbindungen der allgemeinen Formel IC (m = 0), in denen XR¹ Methylamino, R² Wasserstoff, R³ Methyl, R⁴ Methyl und R^{e} Allyl bedeutet und R^{d} für durch R^{x} substituiertes Phenyl steht, wobei R^{x} jeweils einer Zeile der Tabelle B entspricht.

### Tabelle 60

Verbindungen der allgemeinen Formel IC (m = 0), in denen XR¹ Methylamino, R² Methyl, R³ Methyl, R⁴ Methyl und R^{e} Allyl bedeutet und R^{d} für durch R^{x} substituiertes Phenyl steht, wobei R^{x} jeweils einer Zeile der Tabelle B entspricht.

### Tabelle 61

Verbindungen der allgemeinen Formel IC (m = 0), in denen XR¹ Methoxy, R² Wasserstoff, R³ Methyl, R⁴ Methyl und R^{e} trans-Chlorallyl bedeutet und R^{d} für durch R^{x} substituiertes Phenyl steht, wobei R^{x} jeweils einer Zeile der Tabelle B entspricht.

### Tabelle 62

Verbindungen der allgemeinen Formel IC (m = 0), in denen XR¹ Methoxy, R² Methyl, R³ Methyl, R⁴ Methyl und R^{e} trans-Chlorallyl bedeutet und R^{d} für durch R^{x} substituiertes Phenyl steht, wobei R^{x} jeweils einer Zeile der Tabelle B entspricht.

### Tabelle 63

Verbindungen der allgemeinen Formel IC (m = 0), in denen XR¹ Methylamino, R² Wasserstoff, R³ Methyl, R⁴ Methyl und R^{e} trans-Chlorallyl bedeutet und R^{d} für durch R^{x} substituiertes Phenyl steht, wobei R^{x} jeweils einer Zeile der Tabelle B entspricht.

### Tabelle 64

Verbindungen der allgemeinen Formel IC (m = 0), in denen XR¹ Methylamino, R² Methyl, R³ Methyl, R⁴ Methyl und R^{e} trans-Chlorallyl bedeutet und R^{d} für durch R^{x} substituiertes Phenyl steht, wobei R^{x} jeweils einer Zeile der Tabelle B entspricht.

### Tabelle 65

Verbindungen der allgemeinen Formel IC (m = 0), in denen XR¹ Methoxy, R² Wasserstoff, R³ Methyl, R⁴ Methyl und R^{e} Methoxyethyl bedeutet und R^{d} für durch R^{x} substituiertes Phenyl steht, wobei R^{x} jeweils einer Zeile der Tabelle B entspricht.

### Tabelle 66

Verbindungen der allgemeinen Formel IC (m = 0), in denen XR¹ Methoxy, R² Methyl, R³ Methyl, R⁴ Methyl und R^{e} Methoxyethyl bedeutet und R^{d} für durch R^{x} substituiertes Phenyl steht, wobei R^{x} jeweils einer Zeile der Tabelle B entspricht.

### Tabelle 67

Verbindungen der allgemeinen Formel IC (m = 0), in denen XR¹ Methylamino, R² Wasserstoff, R³ Methyl, R⁴ Methyl und R^{e} Methoxyethyl bedeutet und R^{d} für durch R^{x} substituiertes Phenyl steht, wobei R^{x} jeweils einer Zeile der Tabelle B entspricht.

### Tabelle 68

Verbindungen der allgemeinen Formel IC (m = 0), in denen XR¹ Methylamino, R² Methyl, R³ Methyl, R⁴ Methyl und R^{e} Methoxyethyl bedeutet und R^{d} für durch R^{x} substituiertes Phenyl steht, wobei R^{x} jeweils einer Zeile der Tabelle B entspricht.

### Tabelle 69

Verbindungen der allgemeinen Formel IC (m = 0), in denen XR¹ Methoxy und R² Wasserstoff bedeutet und die Kombination der Reste R³, R⁴, R^{d} und R^{e} jeweils einer Zeile der Tabelle C entspricht.

### Tabelle 70

Verbindungen der allgemeinen Formel IC (m = 0), in denen XR¹ Methoxy und R² Methyl bedeutet und die Kombination der Reste R³, R⁴, R^{d} und R^{e} jeweils einer Zeile der Tabelle C entspricht.

### Tabelle 71

Verbindungen der allgemeinen Formel IC (m = 0), in denen XR¹ Methylamino und R² Wasserstoff bedeutet und die Kombination der Reste R³, R⁴, R^{d} und R^{e} jeweils einer Zeile der Tabelle C entspricht.

### Tabelle 72

Verbindungen der allgemeinen Formel IC (m = 0), in denen XR¹ Methylamino und R² Methyl bedeutet und die Kombination der Reste R³, R⁴, R^{d} und R^{e} jeweils einer Zeile der Tabelle C entspricht.

**Tabelle B:**

| Nr. | R^{x} |
|---|---|
| 01 | H |
| 02 | 2-F |
| 03 | 3-F |
| 04 | 4-F |
| 05 | 2,4-F₂ |
| 06 | 2,3-F₂ |
| 07 | 2,4,6-F₃ |
| 08 | 2,3,4,5,6-F₅ |
| 09 | 2-Cl |
| 10 | 3-Cl |
| 11 | 4-Cl |
| 12 | 2,3-Cl₂ |
| 13 | 2,4-Cl₂ |
| 14 | 2,5-Cl₂ |
| 15 | 2,6-Cl₂ |
| 16 | 3,4-Cl₂ |
| 17 | 3,5-Cl₂ |
| 18 | 2,3,4-Cl₃ |
| 19 | 2,3,5-Cl₃ |
| 20 | 2,3,6-Cl₃ |
| 21 | 2,4,5-Cl₃ |
| 22 | 2,4,6-Cl₃ |
| 23 | 3,4,5-Cl₃ |
| 24 | 2,3,4,6-Cl₄ |
| 25 | 2,3,5,6-Cl₄ |
| 26 | 2,3,4,5,6-Cl₅ |
| 27 | 2-Br |
| 28 | 3-Br |
| 29 | 4-Br |
| 30 | 2,4-Br₂ |
| 31 | 2,5-Br₂ |
| 32 | 2,6-Br₂ |
| 33 | 2,4,6-Br₃ |
| 34 | 2,3,4,5,6-Br₅ |
| 35 | 2-I |
| 36 | 3-I |
| 37 | 4-I |
| 38 | 2,4-I₂ |
| 39 | 2-Cl, 3-F |
| 40 | 2-Cl, 4-F |
| 41 | 2-Cl, 5-F |
| 42 | 2-Cl, 6-F |
| 43 | 2-Cl, 3-Br |
| 44 | 2-Cl, 4-Br |
| 45 | 2-Cl, 5-Br |
| 46 | 2-Cl, 6-Br |
| 47 | 2-Br, 3-Cl |
| 48 | 2-Br, 4-Cl |
| 49 | 2-Br, 5-Cl |
| 50 | 2-Br, 6-Cl |
| 51 | 2-Br, 3-F |
| 52 | 2-Br, 4-F |
| 53 | 2-Br, 5-F |
| 54 | 2-Br, 6-F |
| 55 | 2-F, 3-Cl |
| 56 | 2-F, 4-Cl |
| 57 | 2-F, 5-Cl |
| 58 | 4-F, 3-Cl |
| 59 | 5-F, 3-Cl |
| 60 | 4-Br, 3-Cl |
| 61 | 5-Br, 3-Cl |
| 62 | 3-F, 4-Cl |
| 63 | 3-F, 4-Br |
| 64 | 3-Br, 4-Cl |
| 65 | 4-F, 3-Br |
| 66 | 2,6-Cl₂, 4-Br |
| 67 | 2-CH₃ |
| 68 | 3-CH₃ |
| 69 | 4-CH₃ |
| 70 | 2,3-(CH₃)₂ |
| 71 | 2,4-(CH₃)₂ |
| 72 | 2,5-(CH₃)₂ |
| 73 | 2,6-(CH₃)₂ |
| 74 | 3,4-(CH₃)₂ |
| 75 | 3,5-(CH₃)₂ |
| 76 | 2,3,4-(CH₃)₃ |
| 77 | 2,3,5-(CH₃)₃ |
| 78 | 2,3,6-(CH₃)₃ |
| 79 | 2,4,5-(CH₃)₃ |
| 80 | 2,4,6-(CH₃)₃ |
| 81 | 3,4,5-(CH₃)₃ |
| 82 | 2,3,4,6-(CH₃)₄ |
| 83 | 2,3,5,6-(CH₃)₄ |
| 84 | 2,3,4,5,6-(CH₃)₅ |
| 85 | 2-C₂H₅ |
| 86 | 3-C₂H₅ |
| 87 | 4-C₂H₅ |
| 88 | 2,4-(C₂H₅)₂ |
| 89 | 2,6-(C₂H₅)₂ |
| 90 | 3,5-(C₂H₅)₂ |
| 91 | 2,4,6-(C₂H₅)₃ |
| 92 | 2-n-C₃H₇ |
| 93 | 3-n-C₃H₇ |
| 94 | 4-n-C₃H₇ |
| 95 | 2-i-C₃H₇ |
| 96 | 3-i-C₃H₇ |
| 97 | 4-i-C₃H₇ |
| 98 | 2,4-(i-C₃H₇)₂ |
| 99 | 2,6-(i-C₃H₇)₂ |
| 100 | 3,5-(i-C₃H₇)₂ |
| 101 | 2-s-C₄H₉ |
| 102 | 3-s-C₄H₉ |
| 103 | 4-s-C₄H₉ |
| 104 | 2-t-C₄H₉ |
| 105 | 3-t-C₄H₉ |
| 106 | 4-t-C₄H₉ |
| 107 | 4-n-C₉H₁₉ |
| 108 | 2-CH₃, 4-t-C₄H₉ |
| 109 | 2-CH₃, 6-t-C₄H₉ |
| 110 | 2-CH₃, 4-i-C₃H₇ |
| 111 | 2-CH₃, 5-i-C₃H₇ |
| 112 | 3-CH₃, 4-i-C₃H₇ |
| 113 | 2-c-C₆H₁₁ |
| 114 | 3-c-C₆H₁₁ |
| 115 | 4-c-C₆H₁₁ |
| 116 | 2-Cl, 4-C₆H₅ |
| 117 | 2-Br, 4-C₆H₅ |
| 118 | 2-OCH₃ |
| 119 | 3-OCH₃ |
| 120 | 4-OCH₃ |
| 121 | 2-OC₂H₅ |
| 122 | 3-OC₂H₅ |
| 123 | 4-OC₂H₅ |
| 124 | 2-O-n-C₃H₇ |
| 125 | 3-O-n-C₃H₇ |
| 126 | 4-O-n-C₃H₇ |

| Nr. | R^{x} |
|---|---|
| 127 | 2-O-i-C₃H₇ |
| 128 | 3-O-i-C₃H₇ |
| 129 | 4-O-i-C₃H₇ |
| 130 | 2-O-n-C₆H₁₃ |
| 131 | 3-O-n-C₆H₁₃ |
| 132 | 4-O-n-C₆H₁₃ |
| 133 | 2-OCH₂C₆H₅ |
| 134 | 3-OCH₂C₆H₅ |
| 135 | 4-OCH₂C₆H₅ |
| 136 | 2-O(CH₂)₂C₆H₅ |
| 137 | 4-O(CH₂)₂C₆H₅ |
| 138 | 2,3-(OCH₃)₂ |
| 139 | 2,4-(OCH₃)₂ |
| 140 | 2,5-(OCH₃)₂ |
| 141 | 2,6-(OCH₃)₂ |
| 142 | 3,4-(OCH₃)₂ |
| 143 | 3,5-(OCH₃)₂ |
| 144 | 2-O-t-C₄H₉ |
| 145 | 3-O-t-C₄H₉ |
| 146 | 4-O-t-C₄H₉ |
| 147 | 3-(3'-Cl-C₆H₄) |
| 148 | 4-(4'-Cl-C₆H₄) |
| 149 | 2-OC₆H₅ |
| 150 | 3-OC₆H₅ |
| 151 | 4-OC₆H₅ |
| 152 | 2-O-(2 -F-C₆H₄) |
| 153 | 3-O-(3'-Cl-C₆H₄) |
| 154 | 4-O-(4'-CH₃C₆H₄) |
| 155 | 2,3,6-(CH₃)₃, 4-F |
| 156 | 2,3,6-(CH₃)₃, 4-Cl |
| 157 | 2,3,6-(CH₃)₃, 4-Br |
| 158 | 2,4-(CH₃)₂, 6-F |
| 159 | 2,4-(CH₃)₂, 6-Cl |
| 160 | 2,4-(CH₃)₂, 6-Br |
| 161 | 2-i-C₃H₇, 4-Cl, 5-CH₃ |
| 162 | 2-Cl, 4-NO₂ |
| 163 | 4-Cl, 2-NO₂ |
| 164 | 2-OCH₃, 4-NO₂ |
| 165 | 2,4-Cl₂, 5-NO₂ |
| 166 | 2,4-Cl₂, 6-NO₂ |
| 167 | 2,6-Cl₂, 4-NO₂ |
| 168 | 2,6-Br₂, 4-NO₂ |
| 169 | 2,6-I₂, 4-NO₂ |
| 170 | 2-CH₃, 4-Cl, 5-i-C₃H₇ |
| 171 | 2-CO₂CH₃ |
| 172 | 3-CO₂CH₃ |
| 173 | 4-CO₂CH₃ |
| 174 | 2-CH₂OCH₃ |
| 175 | 3-CH₂OCH₃ |
| 176 | 4-CH₂OCH₃ |
| 177 | 2-CH₃, 4-CO-i-C₃H₇ |
| 178 | 2-CH₃, 4-C(CH₃)=NOCH₃ |
| 179 | 2-CH₃, 4-C(CH₃)=NOC₂H₅ |
| 180 | 2-CH₃, 4-C(CH₃)=NO-n-C₃H₇ |
| 181 | 2-CH₃, 4-C(CH₃)=NO-i-C₃H₇ |
| 182 | 2,5-(CH₃)₂, 4-C(CH₃)=NOCH₃ |
| 183 | 2,5-(CH₃)₂, 4-C(CH₃)=NOC₂H₅ |
| 184 | 2,5-(CH₃)₂, 4-C(CH₃)=NO-n-C₃H₇ |
| 185 | 2,5-(CH₃)₂, 4-C(CH₃)=NO-i-C₃H₇ |
| 186 | 2-C₆H₅ |
| 187 | 3-C₆H₅ |
| 188 | 4-C₆H₅ |
| 189 | 2-(2'-F-C₆H₄) |
| 190 | 2-CH₃, 5-Br |
| 191 | 2-CH₃, 6-Br |
| 192 | 3-CH₃, 2-Cl |
| 193 | 4-CH₃, 2-Cl |
| 194 | 5-CH₃, 2-Cl |
| 195 | 3-CH₃, 2-F |
| 196 | 4-CH₃, 2-F |
| 197 | 5-CH₃, 2-F |
| 198 | 3-CH₃, 2-Br |
| 199 | 4-CH₃, 2-Br |
| 200 | 5-CH₃, 2-Br |
| 201 | 3-CH₃, 4-Cl |
| 202 | 3-CH₃, 5-Cl |
| 203 | 3-CH₃, 4-F |
| 204 | 3-CH₃, 5-F |
| 205 | 3-CH₃, 4-Br |
| 206 | 3-CH₃, 5-Br |
| 207 | 4-CH₃, 3-F |
| 208 | 4-CH₃, 3-Cl |
| 209 | 4-CH₃, 3-Br |
| 210 | 4,5-(CH₃)₂, 2-Cl |
| 211 | 4,5-(CH₃)₂, 2-Br |
| 212 | 3,5-(CH₃)₂, 2-Cl |
| 213 | 3,5-(CH₃)₂, 2-Br |
| 214 | 2,6-Cl₂, 4-CH₄ |
| 215 | 2,6-F₂, 4-CH₄ |
| 216 | 2,6-Br₂, 4-CH₄ |
| 217 | 2,4-Br₂, 6-CH₄ |
| 218 | 2,4-F₂, 6-CH₄ |
| 219 | 2,4-Cl₂, 6-CH₄ |
| 220 | 2,6-(CH₃)₂, 4-F |
| 221 | 2,6-(CH₃)₂, 4-Cl |
| 222 | 2,6-(CH₃)₂, 4-Br |
| 223 | 3,5-(CH₃)₂, 4-F |
| 224 | 3,5-(CH₃)₂, 4-Cl |
| 225 | 3,5-(CH₃)₂, 4-Br |
| 226 | 2-CF₃ |
| 227 | 3-CF₃ |
| 228 | 4-CF₃ |
| 229 | 2-OCF₃ |
| 230 | 3-OCF₃ |
| 231 | 4-OCF₃ |
| 232 | 3-OCH₂CHF₂ |
| 233 | 2-NO₂ |
| 234 | 3-NO₂ |
| 235 | 4-NO₂ |
| 236 | 2-CN |
| 237 | 3-CN |
| 238 | 4-CN |
| 239 | 2-CH₃, 3-Cl |
| 240 | 2-CH₃, 4-Cl |
| 241 | 2-CH₃, 5-Cl |
| 242 | 2-CH₃, 6-Cl |
| 243 | 2-CH₃, 3-F |
| 244 | 2-CH₃, 4-F |
| 245 | 2-CH₃, 5-F |
| 246 | 2-CH₃, 6-F |
| 247 | 2-CH₃, 3-Br |
| 248 | 2-CH₃, 4-Br |
| 249 | 2-CH₃, 5-Br |
| 250 | 2-CH₃, 6-Br |
| 251 | 2,5-F₂ |
| 252 | 2,6-F₂ |
| 253 | 3,4-F₂ |
| 254 | 3,5-F₂ |
| n = neo; i = iso; s = sekundär; t = tertiär; c = cyclo | |

**Tabelle C:**

| **Nr.** | **R**^{**3**} | **R**^{**4**} | **R**^{**d**} | **R**^{**e**} |
|---|---|---|---|---|
| 01 | H | CH₃ | CH₃ | CH₃ |
| 02 | CH₃ | CH₃ | CH₃ | CH₃ |
| 03 | C₂H₅ | CH₃ | CH₃ | CH₃ |
| 04 | n-C₃H₇ | CH₃ | CH₃ | CH₃ |
| 05 | i-C₃H₇ | CH₃ | CH₃ | CH₃ |
| 06 | c-C₃H₅ | CH₃ | CH₃ | CH₃ |
| 07 | pyridin-2-yl | CH₃ | CH₃ | CH₃ |
| 08 | pyridin-3-yl | CH₃ | CH₃ | CH₃ |
| 09 | pyridin-4-yl | CH₃ | CH₃ | CH₃ |
| 10 | 5-CH₃-isoxazol-3-yl | CH₃ | CH₃ | CH₃ |
| 11 | phenyl | CH₃ | CH₃ | CH₃ |
| 12 | CH₃ | H | CH₃ | CH₃ |
| 13 | CH₃ | C₂H₅ | CH₃ | CH₃ |
| 14 | CH₃ | n-C₃H₇ | CH₃ | CH₃ |
| 15 | CH₃ | i-C₃H₇ | CH₃ | CH₃ |
| 16 | CH₃ | c-C₃H₅ | CH₃ | CH₃ |
| 17 | CH₃ | pyridin-2-yl | CH₃ | CH₃ |
| 18 | CH₃ | pyridin-3-yl | CH₃ | CH₃ |
| 19 | CH₃ | pyridin-4-yl | CH₃ | CH₃ |
| 20 | CH₃ | 5-CH₃-isoxazol-3-yl | CH₃ | CH₃ |
| 21 | CH₃ | phenyl | CH₃ | CH₃ |
| 22 | CH₃ | CH₃ | H | CH₃ |
| 23 | CH₃ | CH₃ | C₂H₅ | CH₃ |
| 24 | CH₃ | CH₃ | n-C₃H₇ | CH₃ |
| 25 | CH₃ | CH₃ | i-C₃H₇ | CH₃ |
| 26 | CH₃ | CH₃ | c-C₃H₅ | CH₃ |
| 27 | CH₃ | CH₃ | pyridin-2-yl | CH₃ |
| 28 | CH₃ | CH₃ | pyridin-3-yl | CH₃ |
| 29 | CH₃ | CH₃ | pyridin-4-yl | CH₃ |
| 30 | CH₃ | CH₃ | 5-CH₃-isoxazol-3-yl | CH₃ |
| 31 | CH₃ | CH₃ | phenyl | CH₃ |
| 32 | CH₃ | CH₃ | CH₃ | C₂H₅ |
| 33 | CH₃ | CH₃ | CH₃ | n-C₃H₇ |
| 34 | CH₃ | CH₃ | CH₃ | i-C₃H₇ |
| 35 | CH₃ | CH₃ | CH₃ | t-C₄H₉ |
| 36 | CH₃ | CH₃ | CH₃ | benzyl |
| 37 | CH₃ | CH₃ | CH₃ | propargyl |
| 38 | CH₃ | CH₃ | CH₃ | brompropargyl |
| 39 | CH₃ | CH₃ | CH₃ | iodpropargyl |
| 30 | CH₃ | CH₃ | CH₃ | allyl |
| 41 | CH₃ | CH₃ | CH₃ | trans-chlorallyl |
| 42 | CH₃ | CH₃ | CH₃ | CH₂CH₂OCH₃ |
| n = neo; i = iso; s = sekundär; t = tertiär; c = cyclo | | | | |

Ebenso betrifft die vorliegende Erfindung Zwischenprodukte der Formel IV und V 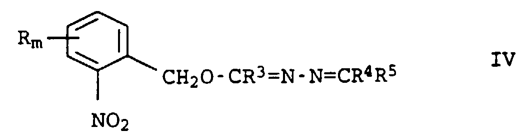 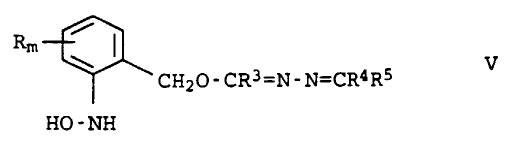 wobei R, R³, R⁴, R⁵ und m die oben genannten Bedeutungen haben.

Die Herstellung von Verbindungen IV und V ist auf Seite 5 bis 7 beschrieben.

Die Verbindungen I eignen sich als Fungizide.

Die Verbindungen I zeichnen sich durch eine hervorragende Wirksamkeit gegen ein breites Spektrum von pflanzenpathogenen Pili zen, insbesondere aus der Klasse der Ascomyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Gemüse, Zierpflanzen, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I eignen sich außerdem zur Bekämpfung von Schadpilzen im Materialschutz (z.B. Holz, Papier, Fasern bzw. Gewebe) und im Vorabschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Schadpilzes bzw. des Einsatzgebiets. Übliche Aufwandmengen sind im Materialschutz beispielsweise 0.001 g bis 2 kg, vorzugsweise 0.005 g bis 1 kg Wirkstoff pro Qubikmeter behandelten Materials.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:

Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;

Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolinacetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4, 5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid, N-Dichlorfluormethylthio-N',N'-dimethyl-N-phenyl-schwefelsäurediamid, 5-Ethoxy-3-trichlormethyl-1,2,4-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-2,2,2-trichloro-1-(4-morpholinyl)ethyl-formamid, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege stictiKalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius Kalifornicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia N-[3-(p-tert.-Butylphenyl)-2-methylpropyl]-cis-2,6-dimethylmorpholin, N-[3- (p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis- (p-chlorphenyl)-3-pyridinmethanol, 1,2-Bis-(3-ethoxycarbonyl-2-thioureido)-benzol, 1,2-Bis-(3-methoxycarbonyl-2-thioureido) -benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethyl-aminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N- (3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus asKalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Kalotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ult-ra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.-% des Wirkstoffs. Die Wirkstoffe werden dabei in einer Reinheit von 90 % bis 100 %, vorzugsweise 95 % bis 100 % (nach NMR-Spektrum) eingesetzt.

### Beispiele für Formulierungen sind:

I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Kalziumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100000 Gew.-Teilen Wasser erhält man eine wäßrige Dispersion, die 0,02 Gew.-% des Wirkstoffs enthält.
VIII.20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Kalzium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1 (nicht erfindungsgemäß)

Herstellung von

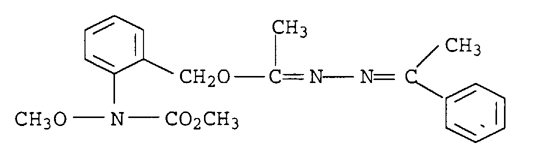

Eine Mischung aus 3,8 g (20 mmol) N-Acetylacetophenon-hydrazon in 15 ml Dimethylformamid wurde portionsweise mit 0,6 g (26 mmol) Natriumhydrid versetzt und solange gerührt, bis die Gasentwicklung beendet war. Die so erhaltene Mischung wurde anschließend mit 8,4 g (20 mmol) N-Methoxy-N-(o-brommethylphenyl)-carbaminsäuremethylester (gemäß WO-A 93/15,046, Reinheit ca. 70 %) versetzt und weitere 12 h bei ca. 25°C gerührt.

Zur Aufarbeitung wurde die Reaktionsmischung mit Wasser versetzt und mehrfach mit tert.-Butyl-methylether extrahiert. Aus den vereinigten organischen Phasen erhielt man nach Waschen mit Wasser, Trocknen und Entfernen des Lösungsmittels das Rohprodukt, welches chromatographisch über eine Säule (Laufmittel: a) Cyclohexan/Methylenchlorid, b) Cyclohexan/Essigsäureethylester) gereinigt wurde. Man erhielt 0,2g (3%) der Titelverbindung als helles Öl.
¹H-NMR (CDCl₃; δ in ppm):
2,2 (s,3H,CH₃); 2,3 (s,3H,CH₃); 3,75 (s,3H, OCH₃); 3,8 (s,3H,OCH₃); 5,35 (s,2H,OCH₂); 7,4 (m,6H,Phenyl); 7,55 (m,1H,Phenyl); 7,85 (m,2H,Phenyl)

### Beispiel 2

Herstellung von

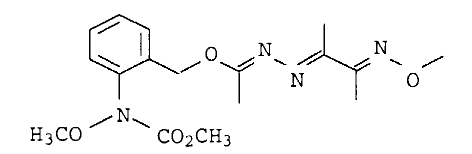

### a) Diacetyl-O-methyloxim-N-acetylhydrazon

Eine Mischung aus 6,0 g (52 mmol) Diacetyl-O-methyloxim, 3,9 g (52 mmol) Acetylhydrazin und 3 Tropfen konzentrierte Salzsäure in 50 ml Methanol wurde über Nacht (ca. 12 Stunden) bei Raumtemperatur (ca. 20°C) gerührt. Das auskristallisierte Produkt wurde isoliert. Die Mutterlauge wurde eingeengt, wobei Reste von Produkt auskristallisierten. Insgesamt erhielt man 4,0 g (45 %) der Titelverbindung. ¹H-NMR (d₆-DMSO; δ in ppm):
1,95 (s,3H,CH₃), 2,00 (s,3H,CH₃), 2,20 (s,3H,CH₃), 3,90 (s,3H,OCH₃), 10,60 (s,breit,1H,NH)

### b) Titelverbindung

Eine Mischung von 1,85 g (11 mmol) Diacetyl-O-methyloxim-N-acetylhydrazon (Beispiel 2a) und 0,29 g (12 mmol) Natriumhydrid in 20 ml Dimethylformamid wurde 15 Minuten bei Raumtemperatur gerührt.
Anschließend gab man 3,3 g (11 mol) N-Methoxy-N-(o-brom-methylphenyl)-carbaminsäuremethylester (gemäß WO-A 93/15046; Reinheit ca. 90 %) hinzu und rührte ca. 1,5 Stunden bei Raumtemperatur.
Anschließend verdünnte man die Reaktionsmischung mit Wasser und extrahierte die wäßrige Phase dreimal mit Methyl-t-butylether. Die vereinigten organischen Phasen wurden mit Wasser gewaschen, über Magnesiumsulfat getrocknet und eingeengt. Der Rückstand wird säulenchromatographisch (Eluent: Cyclohexan/Methyl-t-butylether) gereinigt. Man erhielt 1,3 g (32 %) der Titelverbindung als gelbes Öl.
¹H-NMR (CDCl₃; δ in ppm):
2,05 (s,3H,CH₃), 2,10 (s,3H,CH₃), 3,70 (s,3H,OCH₃), 3,75 (s,3H,OCH₃), 3,95 (s,3H,OCH₃), 5,30 (s,2H,OCH₂), 7,40 (m,3H,Phenyl), 7,55 (m,1H,Phenyl)

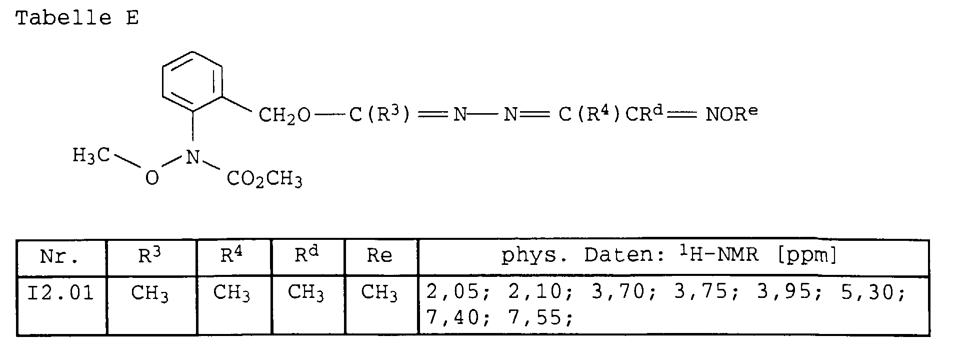

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Puccinia recondita (Weizenbraunrost)

Blätter von Weizensämlingen (Sorte "Kanzler") wurden mit Sporen des Braunrosts *(Puccinia recondita)* bestäubt. Die so behandelten Pflanzen wurden 24 h bei 20 bis 22°C und einer relativen Luftfeuchtigkeit von 90 bis 95 % inkubiert und anschließend mit der wäßrigen Wirkstoffaufbereitung behandelt (Aufwandmenge: 63 pm).

Nach weiteren 8 Tagen bei 20 bis 22°C und 65 bis 70 % relativer Luftfeuchtigkeit wurde das Ausmaß der Pilzentwicklung ermittelt. Die Auswertung erfolgte visuell.

In diesem Test zeigten die mit der Verbindung I2.01 aus Tabelle E behandelten Pflanzen einen Befall von 0 %, während die unbehandelten Pflanzen zu 75 % befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%ige Lösung in Aceton oder
b. als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%ige Hemmung bzw. Mortalität hervorriefen (Wirkschwelle bzw. Minimalkonzentration).

## Patentansprüche

1. Phenylcarbamate der Formel I 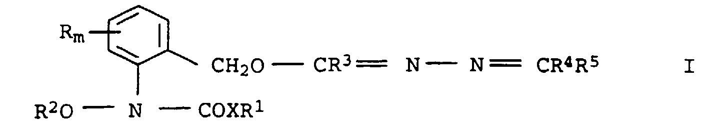 in der die Substituenten und der Index die folgende Bedeutung haben:
R Cyano, Nitro, Trifluormethyl, Halogen, C₁-C₄-Alkyl oder C₁-C₄-Alkoxy;
m 0, 1 oder 2, wobei die Reste R verschieden sein können, wenn n für 2 steht;
R¹ Alkyl, Alkenyl, Alkinyl, Cycloalkyl oder Cycloalkenyl, und für den Fall, daß X für NR^{a} steht, zusätzlich Wasserstoff;
X eine direkte Bindung, O oder NR^{a};
R^{a} Wasserstoff, Alkyl, Alkenyl, Alkinyl, Cycloalkyl oder Cycloalkenyl;
R² Wasserstoff,
ggf. subst. Alkyl, Alkenyl, Alkinyl, Cycloalkyl, Cycloalkenyl, Cycloalkinyl, Alkylcarbonyl oder Alkoxycarbonyl;
R³ und R⁴ unabhängig voneinander
Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können und/oder einen bis drei der folgenden Reste tragen können:
- Cyano, Nitro, Hydroxy, Mercapto, Amino, Carboxyl, Aminocarbonyl, Aminothiocarbonyl,
- C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy,
- C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio und Hetaryl-C₁-C₄-alkylthio, wobei die cyclischen Reste ihrerseits partiell oder vollständig halogeniert sein können und/oder eine bis drei der folgenden Gruppen tragen können: Cyano, Nitro, Hydroxyl, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C (=NOR^{b})-Aₙ-R^{c};
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₅-C₈-cycloalkenyl, C₅-C₈-Cycloalkenyloxy, C₅-C₈-Cycloalkenylthio, C₅-C₈-Cycloalkenylamino, N-C₅-C₈-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Alkoxy-C₁-C₆-alkyl, C₁-C₆-Halogenalkoxy, C₁-C₆-Alkoxycarbonyl, C₁-C₆-Alkylcarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Aryl-C₁-C₆-alkoxy, Aryl, Aryloxy, Hetaryl, Hetaryloxy, wobei die cyclischen Reste der sechs letztgenannten Gruppen partiell oder vollständig halogeniert sein können und/oder eine C₁-C₆-Alkylgruppe tragen können, C(=NOR^{b})-Aₙ-R^{c} oder NR^{f}-CO-D-R^{g};
A Sauerstoff, Schwefel oder Stickstoff, wobei der Stickstoff Wasserstoff oder C₁-C₆-Alkyl trägt;
D eine direkte Bindung, Sauerstoff oder NR^{h};
n 0 oder 1;
R^{b} und R^{c} unabhängig voneinander Wasserstoff oder C₁-C₆-Alkyl;
R^{f} Wasserstoff, Hydroxy, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl;
R^{g}, R^{h} unabhängig voneinander Wasserstoff, C₁-C₆-Alkyl, C₂-C₆-Alkenyl, C₂-C₆-Alkinyl, C₃-C₆-Cycloalkyl, C₃-C₆-Cycloalkenyl, Aryl, Aryl-C₁-C₆-alkyl, Hetaryl und Hetaryl-C₁-C₆-alkyl;
R⁵ eine Gruppe CR^{d}=NOR^{e};
R^{d} eine der bei R³ genannten Gruppen;
R^{e} Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₂-C₁₀-Alkinylcarbonyl oder C₁-C₁₀-Alkylsulfonyl, wobei diese Reste partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die letzten zwölf genannten Gruppen ihrerseits partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR^{b})-Aₙ-R^{c};
Aryl, Arylcarbonyl, Arylsulfonyl, Hetaryl, Hetarylcarbonyl oder Hetarylsulfonyl, wobei diese Reste partiell oder vollständig halogeniert sein können und/oder eine bis drei der folgenden Gruppen tragen können:
- Cyano, Nitro, Hydroxy, Mercapto, Amino, Carboxyl, Aminocarbonyl, Aminothiocarbonyl,
- C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C (=NOR^{b})-Aₙ-R^{c},
sowie deren Salze.

2. Verbindungen der Formel I gemäß Anspruch 1, in denen m für 0 steht.

3. Verbindungen der Formel I gemäß Anspruch 1, in denen R¹ für Methyl steht.

4. Verfahren zur Herstellung der Verbindungen I gemäß Anspruch 1, in denen R³ nicht Halogen bedeutet, dadurch gekennzeichnet, daß man ein Benzylderivat der Formel II 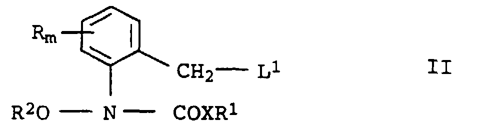 in der L¹ für eine nucleophil austauschbare Abgangsgruppe steht, in an sich bekannter Weise mit einem Carbonsäurehydrazid der Formel III
O=CR³NH―N = CR⁴R⁵ III
umsetzt.

5. Verfahren zur Herstellung der Verbindungen I gemäß Anspruch 1, in denen R² Wasserstoff bedeutet (IA), dadurch gekennzeichnet, daß man ein Nitrobenzol der Formel IV 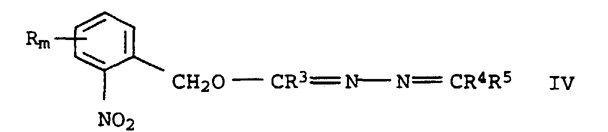 zum entsprechenden Hydroxylamin V 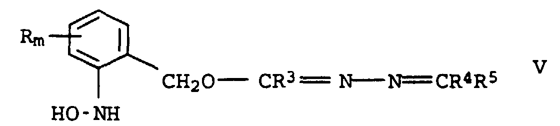 reduziert und V anschließend durch Umsetzung mit einem Acylierungsmittel der Formel VI
L²-COXR¹ VI
in der L² für Halogen oder Aryloxy steht, zu IA umsetzt.

6. Verfahren zur Herstellung der Verbindungen I gemäß Anspruch 1, in denen R² nicht Wasserstoff bedeutet (IB), dadurch gekennzeichnet, daß man eine Verbindungen der Formel IA gemäß Anspruch 5 in an sich bekannter Weise mit einer Verbindung der Formel VII
R²-L³ VII
in der L³ für Halogen, Mesylat, Tosylat, Carboxylat und Sulfat steht, umsetzt.

7. Zwischenprodukte der Formeln IV und V gemäß Anspruch 5, wobei die Reste und der Index die unter Anspruch 1 angegebene Bedeutung haben.

8. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

9. Verfahren zur Bekämpfung von Schadpilzen, dadurch gekennzeichnet, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

10. Verfahren zur Bekämpfung von Schädlingen, dadurch gekennzeichnet, daß man die Schädlingen oder die von ihnen zu schützenden Materialien, Pflanzen, Boden oder Saatgüter mit einer wirksamen Menge einer Verbindung der allgemeinen Formel I gemäß Anspruch 1 behandelt.

## Claims

1. A phenylcarbamate of the formula I 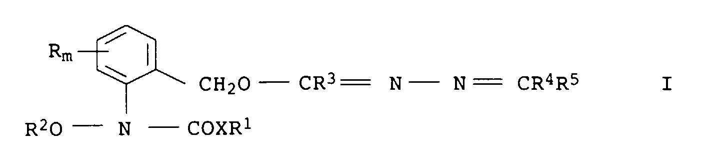 where the substituents and the index have the following meanings:
R is cyano, nitro, trifluoromethyl, halogen, C₁-C₄-alkyl or C₁-C₄-alkoxy;
m is 0, 1 or 2, it being possible for the radicals R to be different if n is 2;
R¹ is alkyl, alkenyl, alkynyl, cycloalkyl or cycloalkenyl, and, in the event that X is NR^{a}, additionally hydrogen;
X is a direct bond, O or NR^{a};
R^{a} is hydrogen, alkyl, alkenyl, alkynyl, cycloalkyl or cycloalkenyl;
R² is hydrogen,
unsubstituted or substituted alkyl, alkenyl, alkynyl, cycloalkyl, cycloalkenyl, cycloalkynyl, alkylcarbonyl or alkoxycarbonyl;
R³ and R⁴ independently of one another are
hydrogen, cyano, nitro, hydroxyl, amino, halogen,
C₁-C₆-alkyl, C₁-C₆-alkoxy, C₁-C₆-alkylthio, C₁-C₆-alkylamino, di-C₁-C₆-alkylamino, C₂-C₆-alkenyl, C₂-C₆-alkenyloxy, C₂-C₆-alkenylthio, C₂-C₆-alkenylamino, N-C₂-C₆-alkenyl-N-C₁-C₆-alkylamino, C₂-C₆-alkynyl, C₂-C₆-alkynyloxy, C₂-C₆-alkynylthio, C₂-C₆-alkynylamino, N-C₂-C₆-alkynyl-N-C₁-C₆-alkylamino, it being possible for the hydrocarbon radicals of these groups to be partially or fully halogenated and/or to have attached to them one to three of the following radicals:
- cyano, nitro, hydroxyl, mercapto, amino, carboxyl, aminocarbonyl, aminothiocarbonyl,
- C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy,
- C₃-C₆-Cycloalkyl, C₃-C₆-cycloalkyloxy, heterocyclyl, heterocyclyloxy, aryl, aryloxy, aryl-C₁-C₄-alkoxy, arylthio, aryl-C₁-C₄-alkylthio, hetaryl, hetaryloxy, hetaryl-C₁-C₄-alkoxy, hetarylthio and hetaryl-C₁-C₄-alkylthio, it being possible for the cyclic radicals, in turn, to be partially or fully halogenated and/or to have attached to them one to three of the following groups: cyano, nitro, hydroxyl, mercapto, amino, carboxyl, aminocarbonyl, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio and C(=NOR^{b})-Aₙ-R^{c};
C₃-C₆-cycloalkyl, C₃-C₆-cycloalkyloxy, C₃-C₆-cycloalkylthio, C₃-C₆-cycloalkylamino, N-C₃-C₆-cycloalkyl-N-C₁-C₆-alkylamino, C₅-C₈-cycloalkenyl, C₅-C₈-cycloalkenyloxy, C₅-C₈-cycloalkenylthio, C₅-C₈-cycloalkenylamino, N-C₅-C₈-cycloalkenyl-N-C₁-C₆-alkylamino, heterocyclyl, heterocyclyloxy, heterocyclylthio, heterocyclylamino, N-heterocyclyl-N-C₁-C₆-alkylamino, aryl, aryloxy, arylthio, arylamino, N-aryl-N-C₁-C₆-alkylamino, hetaryl, hetaryloxy, hetarylthio, hetarylamino, N-hetaryl-N-C₁-C₆-alkylamino, it being possible for the cyclic radicals to be partially or fully halogenated and/or to have attached to them one to three of the following groups:
- cyano, nitro, hydroxyl, mercapto, amino, carboxyl, aminocarbonyl, aminothiocarbonyl,
- C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-alkoxy-C₁-C₆-alkyl, C₁-C₆-haloalkoxy, C₁-C₆-alkoxycarbonyl, C₁-C₆-alkylcarbonyl, C₁-C₆-alkylthio, C₁-C₆-alkylamino, di-C₁-C₆-alkylamino, C₁-C₆- alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, aryl-C₁-C₆-alkoxy, aryl, aryloxy, hetaryl, hetaryloxy, it being possible for the cyclic radicals of the six last-mentioned groups to be partially or fully halogenated and/or to have attached to them a C₁-C₆-alkyl group, or C(=NOR^{b})-Aₙ-R^{c} or NR^{f}-CO-D-R^{g};
A is oxygen, sulfur or nitrogen, the nitrogen having attached to it hydrogen or C₁-C₆-alkyl;
D is a direct bond, oxygen or NR^{h};
n is 0 or 1;
R^{b} and R^{c} independently of one another are hydrogen or C₁-C₆-alkyl;
R^{f} is hydrogen, hydroxyl, C₁-C₆-alkyl, C₂-C₆-alkenyl, C₂-C₆-alkynyl, C₁-C₆-alkoxy, C₂-C₆-alkenyloxy, C₂-C₆-alkynyloxy, C₁-C₆-alkoxy-C₁-C₆-alkyl, C₁-C₆-alkoxy-C₁-C₆-alkoxy and C₁-C₆-alkoxycarbonyl;
R^{g}, R^{h} independently of one another are hydrogen, C₁-C₆-alkyl, C₂-C₆-alkenyl, C₂-C₆-alkynyl, C₃-C₆-cycloalkyl, C₃-C₆-cycloalkenyl, aryl, aryl-C₁-C₆-alkyl, hetaryl and hetaryl-C₁-C₆-alkyl;
R⁵ is a group CR^{d}=NOR^{e};
R^{d} is one of the groups mentioned under R³;
R^{e} is hydrogen,
C₁-C₁₀-alkyl, C₃-C₆-cycloalkyl, C₂-C₁₀-alkenyl, C₂-C₁₀-alkynyl, C₁-C₁₀-alkylcarbonyl, C₂-C₁₀-alkenylcarbonyl, C₂-C₁₀-alkynylcarbonyl or C₁-C₁₀-alkylsulfonyl, it being possible for these radicals to be partially or fully halogenated and/or to have attached to them one to three of the following groups:
- cyano, nitro, hydroxyl, mercapto, amino, carboxyl, aminocarbonyl, aminothiocarbonyl,
- C₁-C₆-alkyl, C₁-C₆-haloalkyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-cycloalkyl, C₃-C₆-cycloalkyloxy, heterocyclyl, heterocyclyloxy, benzyl, benzyloxy, aryl, aryloxy, arylthio, hetaryl, hetaryloxy and hetarylthio, it being possible for the twelve last-mentioned groups, in turn, to be partially or fully halogenated and/or to have attached to them one to three of the following groups:
- cyano, nitro, hydroxyl, mercapto, amino, carboxyl, aminocarbonyl, aminothiocarbonyl,
- C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR^{b})-Aₙ-R^{c};
aryl, arylcarbonyl, arylsulfonyl, hetaryl, hetarylcarbonyl or hetarylsulfonyl, it being possible for these radicals to be partially or fully halogenated and/or to have attached to them one to three of the following groups:
- cyano, nitro, hydroxyl, mercapto, amino, carboxyl, aminocarbonyl, aminothiocarbonyl,
- C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy or c(=NOR^{b})-Aₙ-R^{c},
or a salt thereof.

2. A compound of the formula I as claimed in claim 1 where m is 0.

3. A compound of the formula I as claimed in claim 1 where R¹ is methyl.

4. A process for the preparation of a compound I as claimed in claim 1 where R³ is not halogen, which comprises reacting a benzyl derivative of the formula II 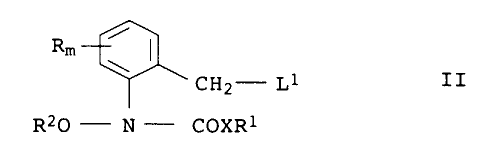 where L¹ is a nucleophilically exchangeable leaving group in a manner known per se with a carbohydrazide of the formula III
O = CR³―NH―N = CR⁴R⁵ III.

5. A process for the preparation of a compound I as claimed in claim 1 where R² is hydrogen (IA), which comprises reducing a nitrobenzene of the formula IV 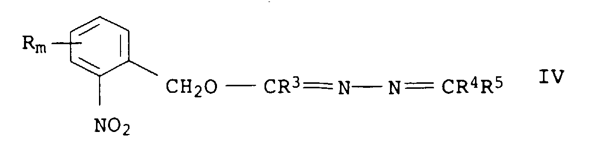 to the corresponding hydroxylamine V 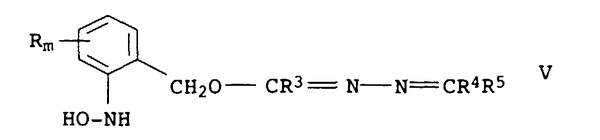 and subsequently reacting V to IA by means of a reaction with an acylating agent of the formula VI
L²-COXR¹ VI
where L² is halogen or aryloxy.

6. A process for the preparation of a compound I as claimed in claim 1 where R² is not hydrogen (IB), which comprises reacting a compound of the formula IA as claimed in claim 5 in a manner known per se with a compound of the formula VII
R²-L³ VII
where L³ is halogen, mesylate, tosylate, carboxylate and sulfate.

7. An intermediate of the formula IV or V as claimed in claim 5 where the radicals and the index have the meanings stated under claim 1.

8. A composition suitable for controlling pests or harmful fungi, comprising a solid or liquid carrier and a compound of the general formula I as claimed in claim 1.

9. A method of controlling harmful fungi, which comprises treating the fungi, or the materials, plants, the soil or seeds to be protected against fungal infection, with an effective amount of a compound of the general formula I as claimed in claim 1.

10. A method of controlling pests, which comprises treating the pests, or the materials, plants, the soil or seeds to be protected from them, with an effective amount of a compound of the general formula I as claimed in claim 1.

## Revendications

1. Phénylcarbamates répondant à la formule I 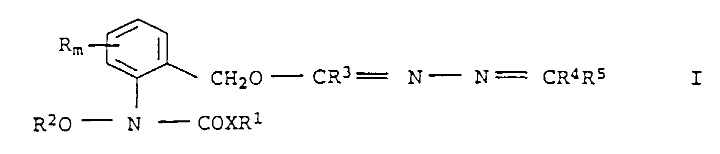 dans laquelle les symboles et l'indice ont les significations suivantes :
R : un groupe cyano, nitro, trifluorométhyle, un halogène, un groupe alkyle en C1-C4 ou alcoxy en C1-C4 ;
m : 0, 1 ou 2, les symboles R pouvant avoir des significations différentes lorsque n est égal à 2 ;
R¹ : un groupe alkyle, alcényle, alcynyle, cycloalkyle ou cycloalcényle, mais en outre l'hydrogène dans le cas où X représente NR^{a} ;
X : une liaison directe, O ou NR^{a} ;
R^{a} : l'hydrogène, un groupe alkyle, alcényle, alcynyle, cycloalkyle ou cycloalcényle ;
R² : l'hydrogène,
un groupe alkyle, alcényle, alcynyle, cycloalkyle, cycloalcényle, cycloalcynyle, alkylcarbonyle ou alcoxycarbonyle éventuellement substitué ;
R³ et R⁴, indépendamment l'un de l'autre :
l'hydrogène, un groupe cyano, nitro, hydroxy, amino, un halogène, un groupe alkyle en C1-C6, alcoxy en C1-C6, alkylthio en C1-C6, alkylamino en C1-C6, di-(alkyle en C1-C6)amino, alcényle en C2-C6, alcényloxy en C2-C6, alcénylthio en C2-C6, alcénylamino en C2-C6, N-(alcényle en C2-C6)-N-alkylamino en C1-C6, alcynyle en C2-C6, alcynyloxy en C2-C6, alcynylthio en C2-C6, alcynylamino en C2-C6, N-(alcynyle en C2-C6)-N-alkylamino en C1-C6, les radicaux hydrocarbonés de ces groupes pouvant être partiellement ou totalement halogénés et/ou pouvant porter un à trois des substituants suivants :
- cyano, nitro, hydroxy, mercapto, amino, carboxyle, aminocarbonyle, aminithiocarbonyle,
- (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alkylsulfonyle en C1-C6, alkylsulfoxyde en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, alcényloxy en C2-C6 ;
- cycloalkyle en C3-C6, cycloalcoxy en C3-C6, hétérocyclyle, hétérocyclyloxy, aryle, aryloxy, aryl-alcoxy en C1-C4, arylthio, aryl-alkylthio en C1-C4, hétéroaryle, hétéroaryloxy, hétéroaryl-alcoxy en C1-C4, hétéroarylthio et hétéroaryl-alkylthio en C1-C4, les radicaux cycliques pouvant eux-mêmes être partiellement ou totalement halogénés et/ou pouvant porter un à trois des groupes suivants : cyano, nitro, hydroxy, mercapto, amino, carboxyle, aminocarbonyle, aminithiocarbonyle, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio et C(=NOR^{b})-Aₙ-R^{c} ;
un groupe cycloalkyle en C3-C6, cycloalcoxy en C3-C6, cycloalkylthio en C3-C6, cycloalkylamino en C3-C6, N-(cycloalkyle en C3-C6)-N-alkylamino en C1-C6, cycloalcényle en C5-C8, cycloalcényloxy en C5-C8, cycloalcénylthio en C5-C8, cycloalcénylamino en C5-C8, N-(cycloalcényle en C5-C8)-N-alkylamino en C1-C6, hétérocyclyle, hétérocyclyloxy, hétérocyclylthio, hétérocyclylamino, N-hétérocyclyl-N-alkylamino en C1-C6, aryle, aryloxy, arylthio, arylamino, N-aryl-N-alkylamino en C1-C6, hétéroaryle, hétéroaryloxy, hétéroarylthio, hétéroarylamino, N-hétéroaryl-N-alkylamino en C1-C6, les radicaux cycliques pouvant être partiellement ou totalement halogénés et/ou pouvant porter un à trois des groupes suivants :
- cyano, nitro, hydroxy, mercapto, amino, carboxyle, aminocarbonyle, aminothiocarbonyle,
- alkyle en C1-C6, halogénoalkyle en C1-C6, alkylsulfonyle en C1-C6, alkylsulfoxyle en C1-C6, cycloalkyle en C3-C6, alcoxy en C1-C6, (alcoxy en C1-C6)alkyle en C1-C6, halogénoalcoxy en C1-C6, (alcoxy en C1-C6)carbonyle, (alkyle en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, aryl-alcoxy en C1-C6, aryle, aryloxy, hétéroaryle, hétéroaryloxy, les radicaux cycliques des six groupes mentionnés en dernier pouvant être partiellement ou totalement halogénés et/ou pouvant porter un groupe alkyle en C1-C6, C(=NOR^{b})-Aₙ-R^{c} ou NR^{f}-CO-D-R^{g} ;
A l'oxygène, le soufre ou l'azote, l'azote portant de l'hydrogène ou un groupe alkyle en C1-C6 ;
D une liaison directe, l'oxygène ou NR^{h} ;
n 0 ou 1 ;
R^{b} et R^{c}, indépendamment l'un de l'autre, l'hydrogène ou un groupe alkyle en C1-C6 ;
R^{f} l'hydrogène, un groupe hydroxy, alkyle en C1-C6, alcényle en C2-C6, alcynyle en C2-C6, alcoxy en C1-C6, alcényloxy en C2-C6, alcynyloxy en C2-C6, (alcoxy en C1-C6)alkyle en C1-C6, (alcoxy en C1-C6)alcoxy en C1-C6 et (alcoxy en C1-C6)carbonyle ;
R^{g}, R^{h}, indépendamment l'un de l'autre, l'hydrogène, un groupe alkyle en C1-C6, alcényle en C2-C6, alcynyle en C2-C6, cycloalkyle en C3-C6, cycloalcényle en C3-C6, aryle, aryl-alkyle en C1-C6, hétéroaryle et hétéroaryl-alkyle en C1-C6 ;
R⁵ un groupe CR^{d}=NOR^{e} ;
R^{d} l'un des groupes mentionnés en référence à R³ ;
R^{e} l'hydrogène,
un groupe alkyle en C1-C10, cycloalkyle en C3-C6, alcényle en C2-C10, alcynyle en C2-C10, (alkyle en C1-C10)carbonyle, (alcényle en C2-C10)carbonyle, (alcynyle en C2-C10)carbonyle ou alkylsulfonyle en C1-C10, ces radicaux pouvant être partiellement ou totalement halogénés et/ou pouvant porter un à trois des groupes suivants :
- cyano, nitro, hydroxy, mercapto, amino, carboxyle, aminocarbonyle, aminothiocarbonyle,
- alkyle en C1-C6, halogénoalkyle en C1-C6, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, cycloalkyle en C3-C6, cycloalcoxy en C3-C6, hétérocyclyle, hétérocyclyloxy, benzyle, benzyloxy, aryle, aryloxy, arylthio, hétéroaryle, hétéroaryloxy et hétéroarylthio, les douze groupes mentionnés en dernier pouvant eux-mêmes être partiellement ou totalement halogénés et/ou pouvant porter un à trois des groupes suivants :
- cyano, nitro, hydroxy, mercapto, amino, carboxyle, aminocarbonyle, aminothiocarbonyle,
- alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio ou C(=NOR^{b})-Aₙ-R^{c} ;
un groupe aryle, arylcarbonyle, arylsulfonyle, hétéroaryle, hétéroarylcarbonyle ou hétéroarylsulfonyle, ces radicaux pouvant être partiellement ou totalement halogénés et/ou pouvant porter un à trois des groupes suivants :
- cyano, nitro, hydroxy, mercapto, amino, carboxyle, aminocarbonyle, aminothiocarbonyle,
- alkyle en C1-C6, halogénoalkyle en C1-C6, (alkyle en C1-C6)carbonyle, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyl_{e,} benzyloxy, aryle, aryloxy, hétéroaryle, hétéroaryloxy ou C(=NOR^{b})-Aₙ-R^{c},
et leurs sels.

2. Composés de formule I selon la revendication 1, dans laquelle m est égal à 0.

3. Composés de formule I selon la revendication 1, dans laquelle R¹ représente un groupe méthyle.

4. Procédé de préparation des composés I selon la revendication 1 dans lesquels R³ ne représente pas un halogène, caractérisé par le fait que l'on fait réagir un dérivé benzylique de formule II 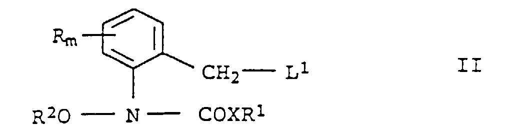 dans laquelle L¹ représente un groupe éliminable par échange nucléophile, de manière connue en soi, avec un hydracide d'acide carboxylique de formule III
O = CR³-NH-N = CR⁴R⁵ III

5. Procédé de préparation des composés I selon la revendication 1 dans lesquels R² représente l'hydrogène (IA), caractérisé par le fait que l'on réduit un nitrobenzène de formule IV 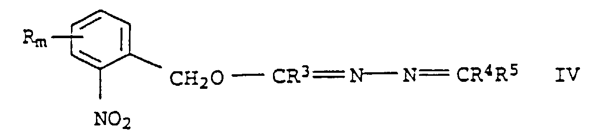 en l'hydroxylamine correspondante V 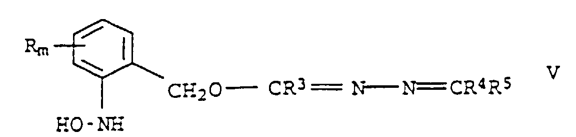 puis on convertit V en IA par réaction avec un agent acylant de formule VI
L² -COXR¹ VI
dans laquelle L² représente un halogène ou un groupe aryloxy.

6. Procédé de préparation des composés I selon la revendication 1 dans lesquels R² ne représente pas l'hydrogène (IB), caractérisé par le fait que l'on fait réagir un composé de formule IA selon la revendication 5, de manière connue en soi, avec un composé de formule VII
R²-L³ VII
dans laquelle L³ représente un halogène, un groupe méthanesulfonate, toluènesulfonate, carboxylate ou sulfate.

7. Produits intermédiaires de formules IV et V selon la revendication V dans lesquelles les symboles et l'indice ont les significations indiquées dans la revendication 1.

8. Produit approprié à l'utilisation pour la lutte contre les parasites ou les mycètes nuisibles, contenant un véhicule solide ou liquide et un composé de formule générale I selon la revendication 1.

9. Procédé pour combattre les mycètes nuisibles, caractérisé par le fait que l'on traite les mycètes ou les matières, végétaux, sols ou semences à protéger contre les mycètes par une quantité efficace d'un composé de formule générale I selon la revendication 1.

10. Procédé pour combattre les parasites, caractérisé par le fait que l'on traite les parasites ou les matières, végétaux, sols ou semences à protéger contre les parasites par une quantité efficace d'un composé de formule générale I selon la revendication 1.
